# EUROPÄISCHE PATENTSCHRIFT

(11) **EP 0 652 216 B1**
(45) Veröffentlichungstag und Bekanntmachung des Hinweises auf die Patenterteilung: **28.02.2001**
(21) Anmeldenummer: 94116720.7
(22) Anmeldetag: 24.10.1994
(51) Int. Cl.: C07D 401/12, A01N 43/54, C07D 407/14, A01N 43/66

(54) **Substituierte Pyridylsalicylaldehyd - bzw. -salicylsäurederivate, Verfahren zu ihrer Herstellung und ihre Verwendung als Herbizide**
Substituted Pyridyl salicylic aldehyde and salicylic acid derivatives, methods for preparing them and their use as herbicides
Dérivés d'aldéhyde salicylique et d'acide salicylique de pyridine, procédés pour leur préparation et leur utilisation comme herbicides

(30) Priorität: 02.11.1993 DE 4337323
(43) Veröffentlichungstag der Anmeldung: 10.05.1995
(73) Patentinhaber: BASF Aktiengesellschaft, 67063 Ludwigshafen (DE)
(72) Erfinder: Rheinheimer, Joachim, Dr., D-67063 Ludwigshafen (DE); Vogelbacher, Uwe Josef, Dr., D-67071 Ludwigshafen (DE); Baumann, Ernst, Dr., D-67373 Dudenhofen (DE); Gerber, Matthias, Dr., D-67117 Limburgerhof (DE); Westphalen, Karl-Otto, Dr., D-67346 Speyer (DE); Walter, Helmut, Dr., D-67283 Obrigheim (DE)

(56) Entgegenhaltungen:
- EP-A- 0 061 913
- EP-A- 0 402 751
- EP-A- 0 435 104
- EP-A- 0 477 637
- EP-A- 0 527 380
- WO-A-91/13065
- WO-A-94/22310
- US-A- 5 149 357
- CHEMICAL ABSTRACTS, vol. 119, no. 15, 11. Oktober 1993, Columbus, Ohio, US; abstract no. 160312g, WADA,N. ET AL. 'Preparation of (carboxyphenoxy)pyrimidines as herbicides' Seite 939 ; & JP-A-05 032 639 (...)

## Beschreibung

Die vorliegende Erfindung betrifft substituierte Pyridylsalicylaldehyd- und -Salicylsäurederivate der allgemeinen Formel I 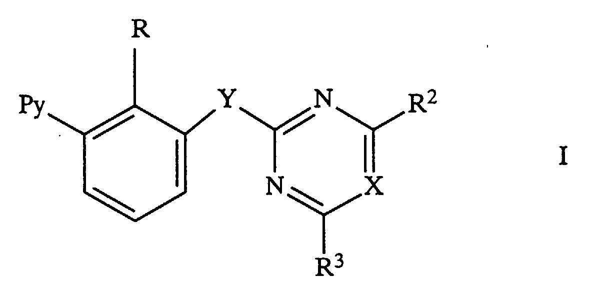 in der R eine Formylgruppe, eine Gruppe CO₂H oder einen zu CO₂H hydrolysierbaren Rest bedeutet und die übrigen Substituenten folgende Bedeutung haben:
- R²: Halogen, C₁-C₄-Alkyl, C₁-C₄-Halogenalkyl, C₁-C₄-Alkoxy, C₁-C₄-Halogenalkoxy oder C₁-C₄-Alkylthio;
- X: Stickstoff oder CR¹³, wobei R¹³ Wasserstoff oder Halogen bedeutet oder zusammen mit R³ eine 3- bis 4-gliedrige Alkylen- oder Alkenylenkette bildet, in der jeweils eine Methylengruppe durch Sauerstoff ersetzt ist;
- R³: Halogen, C₁-C₄-Alkyl, C₁-C₄-Halogenalkyl, C₁-C₄-Alkoxy, C₁-C₄-Halogenalkoxy, C₁-C₄-Alkylthio, oder R³ ist mit R¹³ wie oben angegeben zu einem 5- oder 6-gliedrigen Ring verknüpft;
- Y: Sauerstoff oder Schwefel;
- Py: ein in beliebiger Position verknüpfter Pyridinring, der vier Substituenten R¹⁴, R¹⁵, R¹⁶ und R¹⁷ trägt;
- R¹⁴: a) eine C₁-C₈-Alkylgruppe, welche ein bis fünf Halogenatome und/oder einen der folgenden Reste tragen kann:
   C₁-C₄-Alkoxy, C₁-C₄-Halogenalkoxy, C₁-C₄-Alkylthio, Cyano, C₃-C₈-Cycloalkyl oder Di-C₁-C₄-Alkylamino;
b) eine C₁-C₄-Alkylthiogruppe, welche ein bis fünf Halogenatome und/oder einen der folgenden Reste tragen kann:
   C₁-C₄-Alkoxy, C₁-C₄-Halogenalkoxy, C₁-C₄-Alkylthio, Cyano, C₃-C₈-Cycloalkyl oder Di-C₁-C₄-Alkylamino;
- R¹⁵, R¹⁶, R¹⁷: jeweils unabhängig voneinander Wasserstoff, Nitro, Halogen, C₁-C₈-Alkyl, C₁-C₈-Halogenalkyl sowie die bei R¹⁴ genannten Reste.

Gemäß dem Stand der Technik, z.B. WO 91/13065 und DE-A 39 19 435 weisen Salicylaldehyd- bzw. -Salicylsäurederivate mit einem unsubstituierten Pyridylrest eine herbizide Wirkung auf. In letztgenannter Schrift werden auch entsprechende Verbindungen mit speziell substituierten Pyridylresten wie 6-Methyl-2-pyridyl, 3-Chlor-5-pyridyl und 5-Chlor-2-pyridyl offenbart. Die Wirkung der literaturbekannten Verbindungen ist im Hinblick auf herbizide Wirkung, die bioregulatorische Wirkung und/oder Selektivität nicht immer befriedigend, daher bestand die Aufgabe, pyridylsubstituierte Salicylaldehyd- bzw. -salicylsäurederivate mit verbesserter Wirkung zur Verfügung zu stellen.

Demgemäß wurden die eingangs definierten substituierten Pyridinderivate I gefunden. Die neuen Verbindungen I zeigen eine ausgezeichnete herbizide Wirkung mit zum Teil verbesserter Selektivität gegenüber Kulturpflanzen.

Ferner werden Verfahren und neue Zwischenprodukte zur Herstellung der Verbindungen I sowie ihre Verwendung als Herbizide und Wachstumsregulatoren gefunden.

In der Beschreibung haben die unten genannten Substituenten bevorzugt folgende Bedeutung:
C₁-C₄-Alkyl: Methyl, Ethyl, 1-Propyl, 2-Propyl, 2-Methyl-2 propyl, 2-Methyl-1-propyl, 1-Butyl, 2-Butyl;
C₁-C₈-Alkyl: C₁-C₄-Alkyl sowie Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl;
C₁-C₂-Halogenalkyl: Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor 2-fluorethyl, 2,2,2-Trichlorethyl und Pentafluorethyl;
C₁-C₂-Halogenalkoxy: Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy;
C₁-C₄-Alkoxy: Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
C₁-C₄-Alkylthio: Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
C₃-C₆-Alkenyl: 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
C₃-C₆-Alkinyl: 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-Pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;

Der Rest R ist breit variabel bezüglich der Reste, die sich zur Carboxylgruppe hydrolysieren lassen. Beispielsweise steht R für eine Gruppe 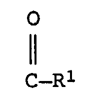 in der R¹ die folgende Bedeutung hat:
a) Wasserstoff;
b) eine Succinylimidoxygruppe;
c) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
   C₁-C₄-Alkyl; C₁-C₄-Halogenalkyl, insbesondere C₁-C₂-Halogenalkyl; C₁-C₄-Halogenalkoxy, insbesondere C₁-C₂-Halogenalkoxy; C₁-C₄-Alkoxy; C₁-C₄-Alkylthio;
d) R¹ ferner ein Rest -(O)ₘ-NR⁶R⁷ in dem m für 0 oder 1 steht und R⁶ und R⁷, die gleich oder unterschiedlich sein können, die folgende Bedeutung haben:
   Wasserstoff
   C₁-C₈-Alkyl, insbesondere C₁-C₄-Alkyl;
   C₃-C₆-Alkenyl, insbesondere 2-Propenyl, 2-Butenyl, 3-Methyl-2-butenyl und 3-Methyl-2-pentenyl;
   C₃-C₆-Alkinyl, vorzugsweise 2-Propinyl, 2-Butinyl, 1-Methyl-2-propinyl und 1-Methyl-2-butinyl, insbesondere 2-Propinyl;
   C₃-C₈-Cycloalkyl, wie Cyclopropyl, Cyclobutyl, Cyclopentyl, Cyclohexyl, Cycloheptyl und Cyclooctyl, wobei diese Alkyl-, Cycloalkyl-, Alkenyl- und Alkinylgruppen jeweils ein bis fünf, insbesondere ein bis drei Halogenatome, bevorzugt Fluor oder Chlor und/oder ein bis zwei der folgenden Gruppen tragen können:
   C₁-C₄-Alkyl, C₁-C₄-Alkoxy, C₁-C₄-Alkylthio, C₁-C₄-Halogenalkoxy, C₃-C₆-Alkenyloxy, C₃-C₆-Alkenylthio, C₃-C₆-Alkinyloxy, C₃-C₆-Alkinylthio, wobei die in diesen Resten vorliegenden Alkenyl- und Alkinylbestandteile vorzugsweise den oben genannten Bedeutungen entsprechen;
   C₁-C₄-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
   C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
   C₃-C₆-Alkenylcarbonyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise wie vorstehend definiert sind;
   Phenyl, gegebenenfalls ein oder mehrfach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
   Di-(C₁-C₄-alkyl)amino wie insbesondere Dimethylamino, Diethylamino, Dipropylamino, N-Propyl-N-methylamino, N-Propyl-N-ethylamino, Diisopropylamino, N-Isopropyl-N-methylamino, N-Isopropyl-N-ethylamino, N-Isopropyl-N-propylamino;
   R⁶ und R⁷ ferner Phenyl, das durch einen oder mehrere der folgenden Reste substituiert sein kann: Halogen, Nitro, Cyano, C₁-C₄-Alkyl, C₁-C₄-Halogenalkyl, insbesondere C₁-C₂-Halogenalkyl, C₁-C₄-Alkoxy, C₁-C₄-Halogenalkoxy, insbesondere C₁-C₂-Halogenalkoxy oder C₁-C₄-Alkylthio;
   oder R⁶ und R⁷ bilden gemeinsam eine zu einem Ring geschlossene, gegebenenfalls substituierte C₄-C₇-Alkylenkette, die ein Heteroatum, ausgewählt aus der Gruppe Sauerstoff, Schwefel oder Stickstoff, enthalten kann wie -(CH₂)₄-, -(CH₂)₅-,-(CH₂)₆-, -(CH₂)₇-, -(CH₂)₂-O-(CH₂)₂-, -CH₂-S-(CH₂)₃-, -(CH₂)₂-O-(CH₃)₃-, NH-(CH₂)₃-, -CH₂-NH-(CH₂)₂-, -CH₂-CH=CH-CH₂-, -CH=CH-(CH₂)₃-, wobei als Substituenten insbesondere C₁-C₄-Alkylreste in Betracht kommen;
e) R¹ ferner eine Gruppe 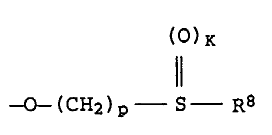 in der k die Werte 0, 1 und 2, p die Werte 1, 2, 3 und 4 annehmen und R⁸ für C₁-C₄-Alkyl, C₁-C₄-Halogenalkyl, C₃-C₆-Alkenyl, C₃-C₆-Alkinyl oder gegebenenfalls substituiertes Phenyl steht, wie insbesondere für R⁶ und R⁷ genannt;
f) R¹ ferner ein Rest OR⁹, worin R⁹ bedeutet:
   i) Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion wie tert.-C₁-C₄-Alkylammonium oder Ammonium [NH₄⁺];
   ii) C₃-C₈-Cycloalkyl wie vorstehend genannt, welches ein bis drei C₁-C₄-Alkylgruppen tragen kann, insbesondere Cyclopropyl, Cyclopentyl, Cyclohexyl, Methylcyclohexyl;
   iii) C₁-C₈-Alkyl, welches ein bis fünf Halogenatome, insbesondere Fluor und Chlor und/oder einen der folgenden Reste tragen kann:
      C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio substituiertes Phenyl oder Phenoxy, wie insbesondere oben genannt;
   iv) eine C₁-C₈-Alkylgruppe, welche ein bis fünf, vorzugsweise ein bis drei Halogenatome, insbesondere Fluor und/oder Chlor tragen kann und einen der folgenden Reste trägt: ein 5-gliedriger Heteroaromat, enthaltend ein bis drei Stickstoffatome oder ein 5-gliedriger Heteroaromat, enthaltend ein Stickstoffatom und ein Sauerstoff- oder Schwefelatom wie Pyrazolyl, Imidazolyl, Benzimidazolyl, Triazolyl, Benztriazolyl, Isooxazolyl, Oxazolyl, Thiazolyl, gebunden über ein C-Atom oder falls möglich N-Atom, wobei der Heteroaromat ein bis vier Halogenatome und/oder ein bis zwei der folgenden Reste tragen kann:
      Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;
   v) eine C₂-C₆-Alkylgruppe, welche in der 2-Position einen der folgenden Reste trägt: C₁-C₄-Alkoxyimino, C₃-C₆-Alkinyloxyimino, C₃-C₆-Halogenalkenyloxyimino oder Benzyloxyimino;
   vi) eine C₃-C₆-Alkenyl- oder eine C₃-C₆-Alkinylgruppe, wobei diese Gruppen ihrerseits ein bis fünf Halogenatome tragen können;
   vii) R⁹ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   viii) ein über ein Stickstoffatom verknüpfter 5-gliedriger Heteroaromat, enthaltend ein bis drei Stickstoffatome wie Pyrazolyl, Imidazolyl, Benzimidazolyl, Triazolyl, Benztriazolyl, vorzugsweise gebunden über die 1-Position, wobei der Heteroaromat ein bis zwei Halogenatome und/oder ein bis zwei der folgenden Reste tragen kann:
      C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
   ix) R⁹ ferner ein Gruppe -N=CR¹⁰R¹¹, worin R¹⁰ und R¹¹, die gleich oder verschieden sein können, bedeuten:
      C₁-C₁₂-Alkyl, insbesondere C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio und/oder einen gegebenenfalls substituierten Phenylrest, wie insbesondere vorstehend genannt, tragen können;
      Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wobei diese Reste insbesondere den oben für R¹ genannten entsprechen;
      oder R¹⁰ und R¹¹ bilden gemeinsam eine C₃-C₁₂-Alkylenkette, welche ein bis drei C₁-C₄-Alkylgruppen tragen und ein Heteroatom aus der Gruppe Sauerstoff, Schwefel und Stickstoff enthalten kann, wie insbesondere bei R⁶ und R⁷ genannt;
   g) R¹ ferner ein Rest -NH-SO₂-R¹² worin R¹² bedeutet:
      C₁-C₄-Alkyl, C₃-C₆-Alkenyl, C₃-C₆-Alkinyl, C₃-C₈-Cycloalkyl wie insbesondere vorstehend für R¹ genannt, wobei diese Reste einen C₁-C₄-Alkoxy-, C₁-C₄-Alkylthio- und/oder einen Phenylrest wie oben genannt tragen können;
      Phenyl, gegebenenfalls substituiert, insbesondere wie vorstehend genannt.
      Besonders bevorzugt steht R¹ für eine Gruppe OR⁹.

Im Hinblick auf die biologische Wirkung sind Wirkstoffe der Formel I bevorzugt, in der die übrigen Substituenten die folgende Bedeutung haben:
- R²: die bei R¹ im einzelnen genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome, insbesondere Chlor, Fluor, Methyl, Methoxy, Ethoxy, Difluormethoxy, Trifluormethoxy, Trifluormethyl, besonders bevorzugt Methoxy;
- X: Stickstoff oder CR¹³, worin
- R¹³: bevorzugt Wasserstoff oder Halogen wie Fluor oder Chlor bedeutet oder zusammen mit R³ eine 4-bis 5-gliedrige Alkylen- oder Alkenylenkette bildet, in der jeweils eine Methylengruppe durch Sauerstoff ersetzt ist, wie -CH₂-CH₂-O-, -CH=CH-O-, -CH₂-CH₂-CH₂-O-, -CH=CH-CH₂O-, insbesondere Wasserstoff, Fluor und -CH₂-CH₂-O-;
- R³: C₁-C₄-Alkyl, C₁-C₄-Halogenalkyl, insbesondere C₁-C₂-Halogenalkyl, C₁-C₄-Alkoxy, C₁-C₄-Halogenalkoxy, insbesondere C₁-C₂-Halogenalkoxy, C₁-C₄-Alkylthiogruppen, insbesondere Fluor, Trifluormethyl, Chlor, Methyl, Methoxy, Ethoxy, Difluormethoxy, Trifluormethoxy, besonders bevorzugt Methoxy, oder mit R¹³ wie oben genannt zu einem 5-oder 6-gliedrigen Ring verknüpft ist;
- Py: ein in 2-, 3- oder 4-Position verknüpfter Pyridinring, der in beliebiger Position vier Substituenten R¹⁴, R¹⁵, R¹⁶ und R¹⁷ trägt;
- R¹⁴: a) eine C₁-C₈-Alkoxygruppe, welche ein bis fünf Halogenatome und/oder einen der folgenden Reste tragen kann:
   C₁-C₄-Alkoxy, C₁-C₄-Halogenalkoxy, besonders C₁-C₂-Halogenalkoxy, C₁-C₄-Alkylthio, Cyano, C₃-C₈-Cycloalkyl oder Di-C₁-C₄-Alkylamino,
   besonders bevorzugt 2-Methoxyethoxy, 2-Ethoxyethoxy, 2-Dimethylaminoethoxy, 2-Diethylaminoethoxy, Methoxymethoxy, Ethoxymethoxy, Dimethylaminomethoxy, Diethylaminomethoxy, 2-Methylthioethoxy, 2,2,2-Trifluorethoxy, Methoxy, Ethoxy, Propoxy, 2-Propoxy;
b) eine C₁-C₄-Alkylthiogruppe, welche ein bis fünf Halogenatome und/oder einen der folgenden Reste tragen kann:
   C₁-C₄-Alkoxy, C₁-C₄-Halogenalkoxy, C₁-C₄-Alkylthio, Cyano, C₃-C₈-Cycloalkyl oder Di-C₁-C₄-Alkylamino, besonders bevorzugt Methylthio, Ethylthio, Propylthio, 2-Propylthio, Methoxymethylthio;
- R¹⁵, R¹⁶, R¹⁷: die unter R¹⁴ genannten Reste sowie Wasserstoff, Nitro; C₁-C₈-Alkyl, C₁-C₂-Halogenalkyl; besonders bevorzugt Wasserstoff, C₁-C₄-Alkyl, Halogen wie insbesondere Fluor oder Chlor, C₁-C₂-Halogenalkyl, C₁-C₄-Alkoxy, C₁-C₂-Halogenalkoxy oder C₁-C₄-Alkylthio.

Besonders bevorzugt sind substituierte Pyridylsalicylaldehyd- und Salicylsäurederivate der Formel I gemäß Anspruch 1, in der mindestens zwei der Reste R¹⁵ bis R¹⁷ Wasserstoff bedeuten.

Ferner sind bevorzugt Verbindungen der Formel I, in der R³ für Methoxy und X für CH oder CF stehen oder in der R³ zusammen mit X eine OCH₂CH₂-Kette bildet.

Aromatische Carbonsäurederivate der Formel I erhält man beispielsweise, indem man ein entsprechendes aromatisches Carbonsäurederivat der Formel II, 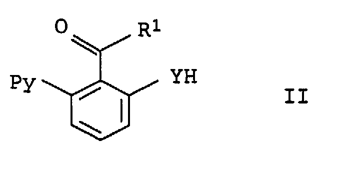 das nach den weiter unten angeführten Beispielen zugänglich ist, mit einer entsprechenden Verbindung der Formel III 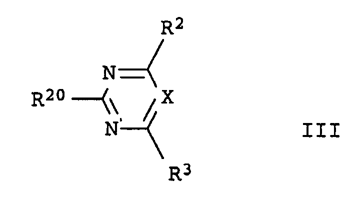 in Gegenwart einer Base umsetzt.

R²⁰ bedeutet Chlor, Brom, Iod, Aryl- oder Alkylsulfonyl wie z. B. Toluolsulfonyl oder Methylsulfonyl oder eine andere äquivalente Abgangsgruppe. Verbindungen der Formel III mit einem reaktionsfähigen Substituenten R²⁰ sind in den meisten Fällen bekannt oder mit dem allgemeinen Fachwissen leicht zu erhalten. Als Base können Alkali- oder Erdalkalimetallhydride wie NaH oder CaH₂, Alkalimetallhydroxyde wie NaOH oder KOH, Alkalimetallalkoholate wie Kalium-tert.-butylat, Alkalimetallcarbonate wie Na₂CO₃ oder K₂CO₃, Alkalimetallamide wie NaNH₂ oder Lithiumdiisopropylamid oder tertiäre Amine wie Triethylamin oder Pyridin Verwendung finden. Bei Einsatz einer anorganischen Base kann man einen Phasentransferkatalysator zusetzen, wenn dies den Umsatz fördert. Dafür kommen beispielsweise Kronenether oder organische Ammoniumverbindungen in Frage.

Es können viele der üblichen Lösungsmittel verwendet werden, wie zum Beispiel Dimethylsulfoxid, Dimethylformamid, Tetrahydrofuran, Methyl-t.-butylether, Diethylether, Aceton, Methylethylketon, Toluol, Benzol, Dimethoxyethan, Dioxan, Pyridin oder t.-Butanol.

Die Reaktionstemperaturen liegen im allgemeinen zwischen -80 und 150°C, vorzugsweise zwischen -10 und 130°C.

Ein weiterer vorteilhafter Herstellungsweg für die Verbindungen I besteht darin, cyclische Acetale der Formel IV 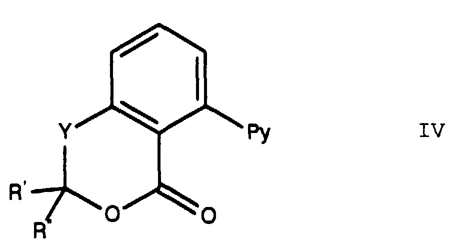 wobei die Substituenten folgende Bedeutung haben:
R', R''
   Wasserstoff;
C₁-C₄-Alkyl, wobei dieser Rest jeweils ein bis fünf Halogenatome und/oder ein bis zwei C₁-C₄-Alkoxy-Gruppen tragen kann;
Phenyl, wobei dieser Rest jeweils ein bis fünf Halogenatome und/oder ein bis zwei der folgenden Gruppen tragen kann: C₁-C₄-Alkyl, C₁-C₄-Alkoxy, C₁-C₄-Halogenalkyl oder Nitro;
ferner die beiden Reste gemeinsam eine C₂-C₆-Alkylenkette, die durch ein bis fünf Halogenatome und/oder C₁-C₄-Alkylreste substituiert sein kann;
Y
   Sauerstoff oder Schwefel;
mit einem Salz der Formel V

R^{1'}-M V

worin M
ein Alkalimetallkation wie Lithium, Natrium, Kalium oder ein Äquivalent eines Erdalkalimetallkations wie Magnesium, Calcium, Barium darstellt und
worin R^{1'} einer Teilmenge von R¹ entspricht und die folgende Bedeutung hat:
   a) ein über ein Stickstoffatom verknüpfter 5-gliedriger Heteroaromat, enthaltend zwei bis drei Stickstoffatome, welcher ein bis zwei Halogenatome und/oder ein bis zwei der folgenden Reste tragen kann:
      C₁-C₄-Alkyl, C₁-C₄-Halogenalkyl, C₁-C₄-Alkoxy, C₁-C₄-Halogenalkoxy und/oder C₁-C₄-Alkylthio;
   b) ein Rest - (O)ₘ-NR⁶R⁷,
      in dem m für 0 oder 1 steht und R⁶ und R⁷, die gleich oder unterschiedlich sein können, die folgende Bedeutung haben:
      Wasserstoff;
      C₁-C₈-Alkyl, C₃-C₆-Alkenyl, C₃-C₆-Alkinyl, C₃-C₈-Cycloalkyl, wobei diese Reste jeweils ein bis fünf Halogenatome und/oder ein bis zwei der folgenden Gruppen tragen können: C₁-C₄-Alkyl, C₁-C₄-Alkoxy, C₃-C₆ Alkenyloxy, C₃-C₆-Alkinyloxy, C₁-C₄-Alkylthio, C₃-C₆-Alkenylthio, C₃-C₆-Alkinylthio, C₁-C₄-Halogenalkoxy, C₁-C₄-Alkylcarbonyl, C₃-C₆-Alkenylcarbonyl, C₃-C₆-Alkinylcarbonyl, C₁-C₄-Alkoxycarbonyl, C₃-C₆-Alkenyloxycarbonyl, C₃-C₆-Alkinyloxycarbonyl, Di-C₁-C₄-alkylamino, C₃-C₈-Cycloalkyl,Phenyl, ein oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl;
      Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
      R⁶ und R⁷ gemeinsam eine zu einem Ring geschlossene, optionell substituierte C₄-C₇-Alkylenkette oder gemeinsam eine zu einem Ring geschlossene, optionell substituierte C₃-C₆-Alkylenkette mit einem Heteroatom, ausgewählt aus der Gruppe Sauerstoff, Schwefel oder Stickstoff;
   c) R¹ ferner eine Gruppe 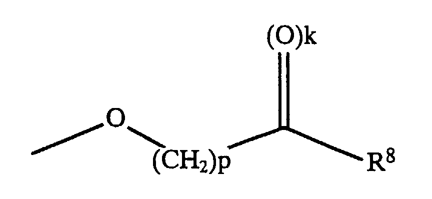 in der R⁸ für C₁-C₄-Alkyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl, C₁-C₄-Halogenalkyl, C₃-C₆-Alkenyl oder C₃-C₆-Alkinyl steht, p die Werte 1, 2, 3 oder 4 und k die Werte 0, 1 oder 2 annehmen;
   d) einen Rest OR⁹, worin R⁹ bedeutet:
      I) eine C₃-C₈-Cycloalkylgruppe, welche ein bis drei C₁-C₄-Alkylreste tragen kann;
      II) eine C₁-C₈-Alkylgruppe, welche ein bis fünf Halogenatome und/oder einen der folgenden Reste tragen kann:
         C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio substituiertes Phenyl oder Phenoxy;
      III) eine C₁-C₈-Alkylgruppe welche ein bis fünf Halogenatome tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welche ein bis vier Halogenatome und/oder ein bis zwei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
      IV) eine C₂-C₆-Alkylgruppe, welche in der 2-Position einen der folgenden Reste trägt: C₁-C₄-Alkoxyimino, C₃-C₆-Alkenyloxyimino, C₃-C₆-Halogenalkenyloxyimino oder Benzyloxyimino;
      V) eine C₃-C₆-Alkenyl- oder eine C₃-C₆-Alkinylgruppe, wobei diese Gruppen ihrerseits ein bis fünf Halogenatome tragen können;
      VI) ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
      VII) ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
      VIII) R⁹ ferner eine Gruppe -N=CR¹⁰R¹¹, worin R¹⁰ und R¹¹, die gleich oder verschieden sind, bedeuten:
         C₁-C₁₂-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio- und/oder einen Phenylrest tragen können;
         Phenyl, das durch einen oder mehrere der folgenden Reste substituiert sein kann:
         Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
         oder R¹⁰ und R¹¹ bilden gemeinsam eine C₃-C₁₂-Alkylenkette, welche ein bis drei C₁-C₄-Alkylgruppen tragen kann;
      e) oder ein Rest -NH-SO₂-R¹², in dem R¹² bedeutet:
         C₁-C₄-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio- und/oder einen Phenylrest tragen können;
         Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio; und dann mit einer Pyridin- bzw. Triazinverbindung der Formel III mit R²⁰ = Halogen wie Fluor, Chlor, Brom, Iod, Alkylsulfonyl, besonders Methylsulfonyl oder Halogenalkylsulfonyl, besonders Trifluormethylsulfonyl, in einem inerten Lösungsmittel umzusetzen. Für R²⁰ kommen die vorstehend beschriebenen Reste in Betracht.
         Diese Umsetzung, die Gegenstand der zeitgleichen deutschen Anmeldung DE-A 4 337 321 ist, kann durch folgendes Reaktionsschema wiedergegeben werden:

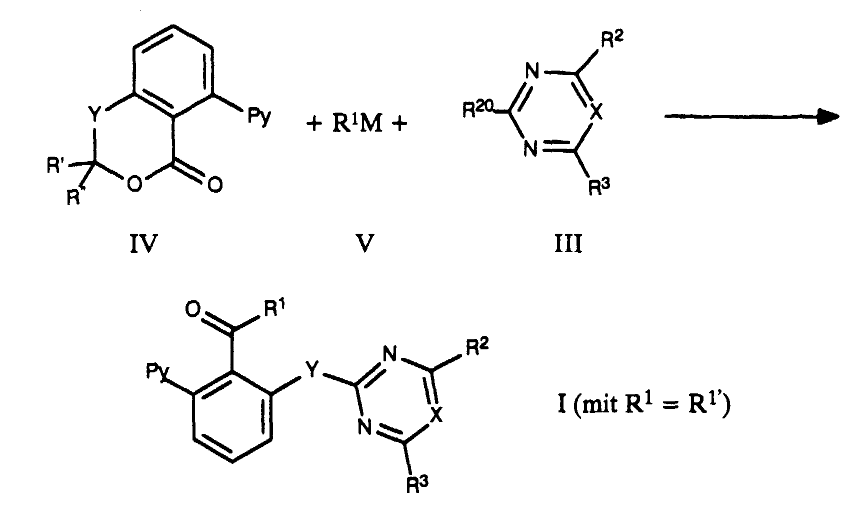

Die Umsetzung der cyclischen Acetale IV mit dem Salz V und die weitere Reaktion mit dem Heterocyclus der Formel III kann in einem Reaktionsgefäß direkt bis zum Wirkstoff I durchgeführt werden. In diesem Fall gibt man zuerst das Salz R¹'M zu, das man auch in situ aus einer Verbindung R¹'H und einer Base herstellen kann. Anschließend fügt man dann den Heterocyclus III hinzu. Die Zugabe von III sollte vorteilhaft erst erfolgen, wenn der erste Schritt der Reaktion, die Addition des Salzes V an das Zwischenprodukt IV, weitgehend abgeschlossen ist. Das kann zwischen wenigen Minuten und etlichen Stunden dauern, wobei die Reaktionstemperatur zwischen -40°C und 200°C, meist zwischen 0°C und 130°C liegt.

Es ist auch möglich, die Reaktion nach der ersten Stufe abzubrechen und das Zwischenprodukt II (mit R¹ = R¹') 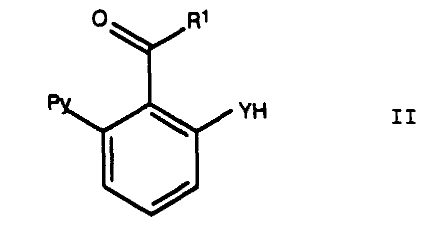 zu isolieren und wie voranstehend beschrieben, weiter umsetzen.

In beiden Fällen sind die üblichen Lösungsmittel geeignet, vorausgesetzt, sie können von der verwendeten Base oder dem Salz R¹'M nicht selbst deprotoniert werden und an der Reaktion teilnehmen.

Besonders gut geeignet sind polare Lösungsmittel, z. B. Ether wie Diethylether, Methyl-tert.-butylether, Dimethoxyethan, Tetrahydrofuran, Dioxan, Amide wie Dimethylformamid, Dimethylacetamid, N-Methylpyrrolidon, Dimethylpropylenharnstoff oder Dimethylsulfoxid. Man kann einen Phasentransfer-Katalysator wie einen Kronenether oder ein quartäres Ammoniumsalz zusetzen, wenn das den Umsatz fördert.

Die Reaktionstemperatur liegt zwischen -40 und 200°C, bevorzugterweise zwischen 0 und 160°C. Die Reaktionszeiten betragen üblicherweise zwischen einigen Minuten und 50 Stunden, meist 0,5-10 Stunden.

Pro Mol Ausgangsstoff IV werden im allgemeinen 0,8 bis 3, insbesondere 0,9 bis 1,5 Moläquivalente der Verbindung V (R¹'M) eingesetzt. Die Menge an Heterocyclus III liegt ebenfalls zweckmäßigerweise bei 0,8 bis 3, insbesondere 0,9 bis 1,5 Moläquivalenten, bezogen auf I.

Die Umsetzung kann kontinuierlich oder diskontinuierlich; bei Atmosphärendruck, Über- oder Unterdruck vorgenommen werden.

Die Möglichkeiten der Aufarbeitung sind vielfältig und hängen im Einzelfall von der Löslichkeit des Produktes in den verwendeten Lösungsmitteln sowie von der Mischbarkeit der Lösungsmittel mit Wasser und den Siedepunkten der Lösungsmittel ab. Sowohl wäßrige als auch nicht-wäßrige Aufarbeitungen sind möglich.

Eine geeignete Aufarbeitungsmethode besteht beispielsweise darin, die Reaktionsmischung, aus der man vorher das Lösungsmittel auch teilweise oder ganz verdampfen kann, mit Wasser zu vermischen und das Produkt aufzufiltrieren oder mit einem organischen Lösungsmittel zu extrahieren.

Die Herstellung der Ausgangsstoffe IV kann vorteilhaft in der Weise erfolgen, daß man 6-Hydroxybenzoesäuren (für Y=0) bzw. 6-Mercaptobenzoesäuren (für Y=S) mit einer Verbindung O=CR'R'' in Gegenwart eines sauren Katalysators umsetzt: 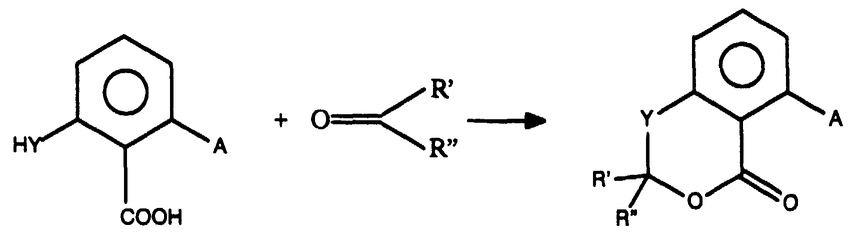 Dabei bedeutet A ein Halogenatom, z.B. Chlor, Brom, Iod, C₁-C₄-Halogenalkylsulfonyloxy, insbesondere Trifluormethylsulfonyloxy, C₁-C₄-Alkylsulfonyloxy oder Fluorsulfonyloxy.

Die Ausgangsstoffe sind allgemein bekannt oder in allgemein bekannter Weise zugänglich.

Man kann zur Bindung des bei der Reaktion entstehenden Wassers ein wasserentziehendes Mittel zusetzen wie z.B. Molsieb, Säureanhydride, wie Trifluoracetanhydrid, Acetanhydrid, Natriumsulfat, Calciumchlorid, wenn das den Umsatz fördert. Geeignet ist auch ein reaktionsfähiges Acetal der Verbindung O=CR'R", wie z.B. ein Dimethylacetal, Diethylacetal, Ethylenacetal oder Propylenacetal. Als Katalysatoren eignen sich besonders saure Ionenaustauscher, Protonensäuren wie z.B. Toluolsulfonsäure, Schwefelsäure, Phosphorsäure usw. oder Lewis-Säuren wie z.B. Aluminiumchlorid, Titantetrachlorid, Zinkchlorid, Calciumchlorid, Magnesiumchlorid, Zinnchloride usw. Oftmals lassen sich das gebildete Wasser oder der aus dem Acetal gebildete Alkohol direkt aus der Reaktionsmischung abdestillieren.

Die weitere Umsetzung zu den cyclischen Acetalen IV erfolgt mit Stannyl- oder Boranylverbindungen der Formel W-Py in Gegenwart einer katalytisch wirksamen Palladiumverbindung gemäß folgendem Reaktionsschema: 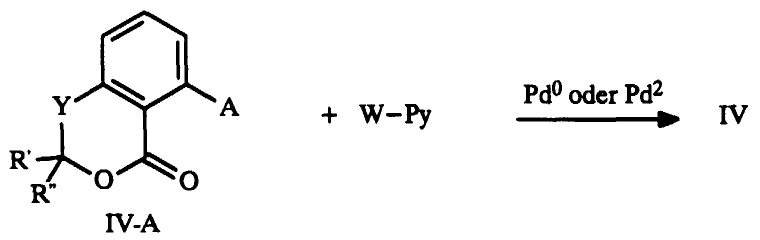 W bedeutet Trialkylstannyl, z.B. Tri-C₁-C₈-alkylstannyl wie Trimethylstannyl, Triethylstannyl, Tripropylstannyl, Tributylstannyl, Tripentylstannyl oder Trihexylstannyl, Dihydroxyboranyl, Dialkoxyboranyl, z.B. Di-C₁-C₄-alkoxyboranyl wie Dimethoxyboranyl, Diethoxyboranyl, Dipropoxyboranyl, Diisopropoxyboranyl oder Dibutoxyboranyl oder Isomere oder Alkylendioxyboranyl, z.B. C₁-C₄-Alkylendioxyboranyl wie Ethylendioxyboranyl oder 1,3-Propylendioxyboranyl. Die verwendeten Bor- oder Zinnverbindungen sind entweder bekannt oder analog zu bekannten Verbindungen herstellbar.

Bei diesem neuen Verfahren wird eine katalytisch wirksame Palladiumverbindung eingesetzt. Dabei sind beliebige Palladiumsalze oder -Komplexe geeignet, die in der Reaktionsmischung zumindest teilweise löslich sind. Die Oxidationsstufe des Palladiums kann 0 oder 2 betragen. Bei den Palladiumsalzen kommen u. a. folgende Gegenionen in Betracht: Chlorid, Bromid, Iodid, Sulfat, Acetat, Trifluoracetat, Acetylacetonat oder Hexafluoro-2,4-pentadionat. Es können viele verschiedene Palladiumkomplexe verwendet werden. Voraussetzung ist lediglich, daß die Liganden am Palladium unter den Reaktionsbedingungen vom Substrat verdrängt werden können. Besonders geeignet sind Phosphinliganden wie z. B. Aryl-Alkylphosphine wie u. a. Methyldiphenylphosphin, Isopropyldiphenylphosphin, Triarylphosphine wie u. a. Triphenylphosphin, Tritolylphosphin, Trixylylphosphin, Trihetarylphosphine wie Trifurylphosphin oder dimere Phosphine. Gut geeignet sind auch olefinische Liganden wie u. a. Dibenzylidenaceton oder seine Salze, Cycloocta-1,5-dien oder Amine wie Trialkylamine (z. B. Triethylamin, Tetramethylethylendiamin, N-Methylmorpholin) oder Pyridin.

Man kann den verwendeten Komplex direkt bei der Reaktion einsetzen. So kann man z. B. mit Tetrakistriphenylphosphinpalladium(0), Bistriphenylphosphinpalladiumdichlorid, Bistriphenylphosphinpalladiumdiacetat, einem Dibenzylidenaceton-Palladium(0)-Komplex, Tetrakismethyldiphenylphosphinpalladium(0) oder Bis(1,2-diphenylphosphinoethan)palladiumdichlorid verfahren. Man kann auch ein Palladiumsalz und zusätzlich einen geeigneten Liganden verwenden, die dann erst in situ den katalytisch aktiven Komplex bilden. Diese Vorgehensweise bietet sich z. B. bei den oben genannten Salzen und Phosphinliganden wie z. B. Trifurylphosphin oder Tritolylphosphin an. Auch können Palladiumkomplexe wie z. B. Tris(dibenzylidenaceton)dipalladium, Bis(dibenzylidenaceton)palladium oder 1,5-Cyclooctadienpalladiumdichlorid durch die Zugabe von Liganden wie z. B. Trifurylphosphin oder Tritolyli phosphin weiter aktiviert werden.

Üblicherweise werden 0,001 bis 10 mol-%, insbesondere 0,005 bis 5 mol-% der Palladiumverbindung (Salz oder Komplex), bezogen auf die Verbindung W-Py verwendet. Höhere Mengen sind möglich, aber eher unwirtschaftlich.

Die Menge an W-Py, bezogen auf den Ausgangsstoff IV-A, liegt im allgemeinen bei 0,8 bis 3, bevorzugt 0,95 bis 1,5 Moläquivalenten.

Für die Reaktion sind alle Lösungsmittel geeignet, die nicht selbst mit den verwendeten Substraten reagieren. Polare Lösungsmittel beschleunigen die Reaktion. Besonders geeignet sind Ether wie Diethylether, Methyl-tert.-butylether, Dimethoxyethan, Tetrahydrofuran, Dioxan, Amide wie Dimethylformamid, Dimethylacetamid, N-Methylpyrrolidon, Dimethylpropylenharnstoff oder Amine wie Triethylamin. Vorteilhaft ist oftmals die Verwendung von Mischungen z. B. von Ethern mit Amiden oder Mischungen der obengenannten Lösungsmittel mit Wasser oder aliphatischen Alkoholen. Die Zugabe von Tetraalkylammoniumhalogeniden oder Alkalimetallhalogeniden wie z. B. Lithiumchlorid ist oft hilfreich und insbesondere anzuraten, wenn A für Sulfonyloxireste steht.

Die Reaktionstemperatur liegt zwischen -20 und 200°C, bevorzugterweise zwischen 50 und 160°C. Die Reaktionszeiten betragen üblicherweise zwischen einigen Minuten und 50 Stunden, meist 0,5-10 Stunden. Bei der Verwendung niedrig siedender Lösungsmittel ist es manchmal nützlich, die Umsetzung unter Eigendruck im Autoklaven durchzuführen.

Soweit es sich bei den in der beschriebenen Weise hergestellten Verbindungen der Formel I um Carbonsäuren handelt (wenn also R¹ Hydroxyl bedeutet), können hieraus die im Anspruch 2 aufgeführten Carbonsäurederivate z. B. auch dadurch hergestellt werden, daß man die Carbonsäure zunächst auf übliche Weise in eine aktivierte Form wie ein Halogenid oder Imidazolid überführt und dieses dann mit der entsprechenden Hydroxylverbindung der Formel R¹-H umsetzt. Diese beiden Schritte lassen sich beispielsweise auch dadurch vereinfachen, daß man die Carbonsäure in Gegenwart eines wasserabspaltenden Mittels wie z. B. eines Carbodiimids oder eines geeigneten Phosphonsäureanhydrids auf die Hydroxylverbindung einwirken läßt.

Weiterhin lassen sich Carbonsäuren der Formel I (wobei also R¹ Hydroxyl bedeutet) auch wie folgt zu vielen der beschriebenen Ester umsetzen: Man überführt die Carbonsäure dazu zunächst in ein Salz - besonders ein Alkalimetallsalz - und bringt dieses dann - gegebenenfalls in Gegenwart einer der weiter oben genannten Basen - mit einem Alkylierungsmittel zur Reaktion. Das Alkylierungsmittel hat die allgemeine Formel R¹-R²¹, wobei für R²¹ die üblichen Abgangsgruppen, wie beispielsweise Chlor, Brom, Iod, Aryl- oder Alkylsulfonyl wie z. B. Toluolsulfonyl oder Methylsulfonyl in Betracht kommen. Die verwendeten Alkylierungsmittel sind im allgemeinen bekannt oder sie lassen sich analog zu bekannten Verbindungen herstellen.

Die Verbindungen I bzw. die sie enthaltenden Mittel sowie deren umweltverträgliche Salze z.B. von Alkalimetallen und Erdalkalimetallen können als Herbizide in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 bis 95 Gew.-%, vorzugsweise zwischen 0,5 bis 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können weiterhin beispielsweise wie folgt formuliert werden:
I. 20 Gew.-Teile der Verbindung Nr. 1.092 werden in einer Mischung gelöst, die aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.
II. 20 Gew.-Teile der Verbindung Nr. 1.092 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenyl und 10 Gew.-Teilen des Anlagerungsproduktes 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
III. 20 Gew.-Teile des Wirkstoffs Nr. 1.092 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinus besteht. Durch Eingießen und feines Verteilen der Lösung in 100 000 Gew.-Teilen Wasser erhält man eine wäßrige Dispersion, die 0,02 Gew.-% des Wirkstoffs enthält.
IV. 20 Gew.-Teile des Wirkstoffs Nr. 1.6 werden mit 3 Gew.-Teilen des Natriumsalzes der Diisobutylnaphthalin-α-sulfonsäure, 17 Gew.-Teile des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.
V. 3 Gew.-Teile des Wirkstoffs Nr. 1.092 werden mit 97 Gew.-Teilen feinteiligem Kaolin vermischt. Man erhält auf diese Weise ein Stäubemittel, das 3 Gew.-% des Wirkstoffs enthält.
VI. 20 Gew.-Teile des Wirkstoffs Nr. 1.092 werden mit 2 Gew.Teilen Calciumsalz der Dodecylbenzolsulfonsäure, 8 Gew.Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile Ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 2,0, vorzugsweise 0,01 bis 1,0 kg/ha aktive Substanz (a.S.). In Anbetracht der Vielseitigkeit der Applikationsmethoden können die erfindungsgemäßen Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius , Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays,.

Die Verbindungen der Formel I können weiterhin die verschiedenen Entwicklungsstadien einer Pflanze beeinflussen und deshalb als Wachstumsregulatoren eingesetzt werden. Die Wirkungsvielfalt der Pflanzenwachstumsregulatoren ist vor allem abhängig von der Pflanzenart und -sorte; von dem Zeitpunkt der Applikation, bezogen auf das Entwicklungsstadium der Pflanze, und von der Jahreszeit; von dem Applikationsort und -verfahren (z.B. Samenbeize, Bodenbehandlung, Blattapplikation oder Stamminjektion bei Bäumen); von klimatischen Faktoren (z.B. Temperatur, Niederschlagsmenge, außerdem Tageslänge und Lichtintensität); von der Bodenbeschaffenheit (einschließlich Düngung); von der Formulierung bzw. Anwendungsform des Wirkstoffs und von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt:
A.
   Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.
   Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.
B.
   Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zukkerrohr sowie Zitrusfrüchte zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C.
   Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.
D.
   Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es nämlich zu einer besseren Ausnutzung des vorhandenen Wassers, weil u.a. die Öffnungsweite der Stomata reduziert wird, eine dickere Epidermis und Cuticula ausgebildet werden, die Durchwurzelung des Bodens verbessert wird und das Mikroklima im Pflanzenbestand durch einen kompakteren Wuchs günstig beeinflußt wird.
   Besonders gut eignen sich die Verbindungen I zur Halmverkürzung von Kulturpflanzen wie Gerste, Raps und Weizen.
   Die erfindungsgemäß zu verwendenden Wachstumsregulatoren der Formel I können den Kulturpflanzen sowohl von Samen her (als Saatgutbeizmittel) als auch über den Boden, d.h. durch die Wurzel sowie - besonders bevorzugt - durch Spritzung über das Blatt, zugeführt werden. Die Herstellung der Mittel erfolgt dabei analog zu der von Herbiziden (s.o.).
   Die Aufwandmenge an Wirkstoff ist infolge der hohen Pflanzenverträglichkeit nicht kritisch. Die optimale Aufwandmenge variiert ja nach Bekämpfungsziel, Jahreszeit, Zielpflanzen und Wachstumsstadien.
   Zur Verbreiterung des Wirkungsspektrums und zur Erzielung synergistischer Effekte können die Pyridylsalicylaldehyd- und Salicylsäurederivate I mit zahlreichen Vertretern anderer herbizider oder wachstumsregulierender Wirkstoffgruppen gemischt und gemeinsam ausgebracht werden. Beispielsweise kommen als herbizide Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dion-derivate, die in 2-Stellung z.B. eine Carboxy- oder Carbimino-Gruppe tragen, Chinolincarbonsäurederivate, Imidazoline, Sulfonamide, Sulfonylharnstoffe, Aryloxy-, Heteroaryloxyphenoxypropionsäuren sowie deren Salze, Ester und Amide und andere in Betracht. Bei Wachstumsregulatoren kommen insbesondere Chlormequatchlorid, Ethylen und Mepiquatchlorid in Frage.
   Außerdem kann es von Nutzen sein, die Verbindungen I allein oder in Kombination mit anderen Herbiziden oder Wachstumsregulatoren auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Synthesebeispiele

### Beispiel 1

a) 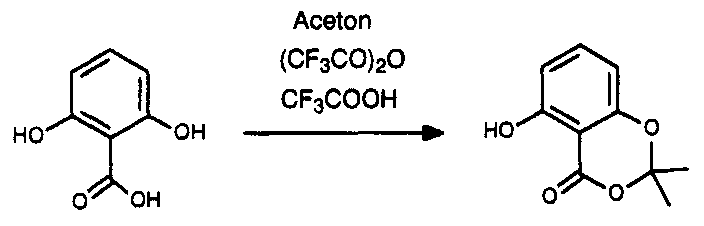 5-Hydroxy-2,2-dimethyl-4H-(1,3)benzodioxin-4-on
   Man legt 100 g (0,66 Mol) 2,6-Dihydroxybenzoesäure in 800 ml Trifluoressigsaure vor und gibt 100 ml Aceton sowie 216 g (1,98 Mol) Trifluoracetanhydrid zu. Man kocht ca. 2 h unter Rückfluß, und tropft anschließend unter Rückfluß 50 ml Aceton pro Stunde zu (gesamte Reaktionszeit 7,5 h, gesamte Acetonzugabe 375 ml). Die Reaktionsmischung wird im Vakuum bei ca. 55°C eingeengt, dreimal mit Toluol aufgefüllt und wieder eingeengt und schließlich bei 45°C an der Ölpumpe getrocknet.
   Das ölige Produkt wird in 2 l Methyl-tert.-butylether aufgenommen, mit 2 l Wasser und 2 l gesättigter NaHCO₃-Lösung versetzt und 1,5 h gerührt. Man trennt ab, extrahiert mit Methyl-tert.-butylether, wäscht die vereinigten organischen Phasen mit Kochsalzlösung, trocknet über Natriumsulfat und engt ein. Der Rückstand wird mit 200 ml n-Pentan verrührt, abgesaugt und getrocknet. Ausbeute: 115 g (90%). Schmp. 60-62°C.
b) 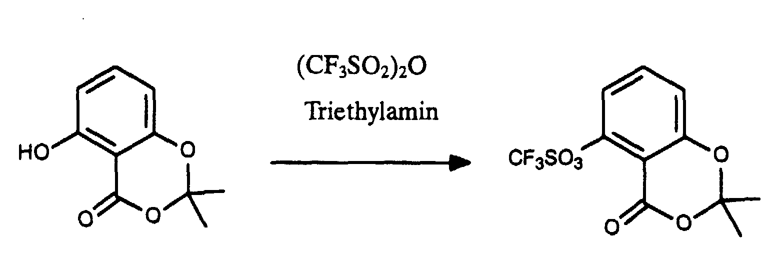 2,2-Dimethyl-5-trifluormethylsulfonyloxy-4H-(1,3)benzodioxin-4-on:
   Man löst 80 g (0,41 Mol) 5-Hydroxy-2,2-dimethyl-4H-(1,3)benzodioxin-4-on in 1,5 l Methylenchlorid, gibt bei 0°C 129 g (1,28 Mol) Triethylamin zu und tropft dann innerhalb von 2 h 314 g (1,11 Mol) Trifluormethylsulfonsäureanhydrid zu. Man läßt auf 10°C erwärmen, rührt bei dieser Temperatur 10 min nach und gibt die Mischung dann unter Rühren in 1,5 l Wasser bei 0°C. Man trennt die organische Phase ab, extrahiert mit Methylenchlorid, wäscht die vereinigten organischen Phasen mit Wasser und einmal mit gesättigter Kochsalzlösung, trocknet über Natriumsulfat und engt ein. Der Rückstand wird mit 200 ml n-Pentan verrührt, abfiltriert, mit n-Pentan nachgewaschen und bei 40°C an der Ölpumpe getrocknet. Ausbeute: 118 g (88 %). Schmelzpunkt: 115°C.
c) 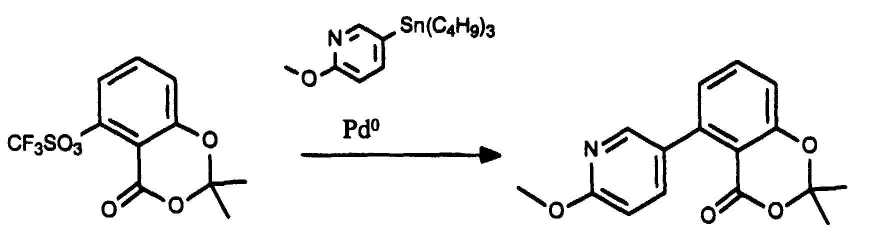 2,2-Dimethyl-5-(2-methoxypyridin-5-yl)-4H-(1,3)benzodioxin-4-on
   Man löst 8,7 g 2,2-Dimethyl-5-trifluormethylsulfonyloxy-4H-(1, 3)benzodioxin-4-on, 23,2 g 2-Methoxy-5-tributylstannylpyridin, 3,4 g Lithiumchlorid, 0,6 g Tetrakistriphenylphosphinpalladium⁰ und 70 mg 2,6-Di-t.-butyl-4-methylphenol in 150 ml Dioxan und rührt 3 h bei 140°C im Autoklaven. Man engt im Vakuum ein, verrührt mit 200 ml n-Pentan, filtriert über wenig Kieselgel-60, wäscht mit n-Pentan nach und eluiert das Produkt mit Essigsäureethylester. Nach dem Einengen verbleiben 10,3 g vom Schmp. 160°C.
d)
   2-(4,6-Dimethoxypyrimidin-2-yloxy)-6-(2-methoxypyridin-5-yl)benzoesäureacetonoximester (Verbindung 2.008)
   0,67 g Acetonoxim, gelöst in 30 ml Toluol werden mit 1,66 g einer 30 %igen Natriummethylat-Lösung in Methanol versetzt und im Vakuum eingeengt. Man gibt 35 ml Dimethylformamid und dann 2,50 g 2,2-Dimethyl-5-(2-methoxypyridin-5-yl)-4H-(1,3)benzodioxin-4-on zu, rührt 15 min bei Raumtemperatur und fügt schließlich 1,90 g 4,6-Dimethoxy-2-methylsulfonylpyrimidin hinzu. Man rührt mehrere Stunden nach, gießt das Reaktionsgemisch in 300 ml Wasser, extrahiert mit Methyl-t.-butylether, wäscht gründlich mit Wasser und gesättigter Kochsalzlösung, trocknet über Natriumsulfat und engt im Vakuum ein. Das Rohprodukt wird durch Chromatographie an Kieselgel-60 mit n-Hexan/Essigsäureethylester gereinigt. ¹H-NMR (CDCl₃): s. Tabelle 2.

### Beispiel 2

a)
   2,2-Dimethyl-5-(6-methoxypyridin-2-yl)-4H-(1,3)benzodioxin-4-on
   Man löst 10,9 g 2,2-Dimethyl-5-trifluormethylsulfonyloxy-4H-(1, 3)benzodioxin-4-on, 16,0 g 6-Methoxy-2-tributylstannylpyridin, 4,25 g Lithiumchlorid, 0,78 g Tetrakistriphenylphosphinpalladium⁰ und 40 mg 2,6-Di-t.-butyl-4-methylphenol in 120 ml Dioxan und rührt 4 h bei 140°C im Autoklaven. Man engt im Vakuum ein und chromatographiert das Produkt an Kieselgel-60 mit Essigsäureethylester/Toluol. Man erhält 9,1 g (95 %) eines Feststoffes vom Schmp. 130-132°C.
b)
   2-(4,6-Dimethoxypyrimidin-2-yloxy)-6-(6-methoxypyridin-2-yl)-benzoesäurebenzylester (Verbindung 1.012)
   1,36 g Benzylalkohol gelöst in 50 ml Dimethylformamid werden mit 380 mg Natriumhydrid (80 %ig in Paraffinöl) versetzt und ca. 1 h bei Raumtemperatur nachgerührt. Man gibt 3,0 g 2,2-Dimethyl-5-(6-methoxypyridin-2-yl)-4H-(1,3)benzodioxin-4-on zu, rührt 3 h bei Raumtemperatur und fügt schließlich 2,65 g 4,6-Dimethoxy-2-methylsulfonylpyrimidin hinzu. Man rührt 2 h bei Raumtemperatur, 1,5 h bei 80°C und 3 h bei 115°C, gießt in phosphorsaures Eiswasser, extrahiert mit Essigsäureethylester, wäscht mit Wasser, trocknet über Natriumsulfat und engt im Vakuum ein. Das Rohprodukt wird durch Chromatographie an Kieselgel-60 mit Cyclohexan/Toluol/Essigsäureethylester gereinigt. ¹H-NMR (CDCl₃): s. Tabelle 1.

### Beispiel 3

2-(4,6-Dimethoxypyrimidin-2-yloxy)-6-(6-methoxypyridin-2-yl)benzoesäure (Verbindung 1.004)

1,57 g 2-(4,6-Dimethoxypyrimidin-2-yloxy)-6-(6-methoxypyridin-2-yl)-benzoesäurebenzylester und 560 mg Palladium auf Aktivkohle (10 %) in 100 ml Diethylether werden bei Raumtemperatur 10 h mit 50 bar Wasserstoffdruck hydriert. Man gibt dann nochmals 300 mg Katalysator zu und hydriert noch 10 h bei Raumtemperatur mit 50 bar Wasserstoffdruck. Es wird über Celite® (Fa. Aldrich) filtriert und im Vakuum eingeengt. Ausbeute: 0,99 g eines Feststoffes vom Schmp. 158-160°C.

Besonders bevorzugte Verbindungen der Formel I, die gemäß den vorausstehenden Beispielen herstellbar sind, sind in nachfolgenden Tabellen 1 bis 6 zusammengestellt.

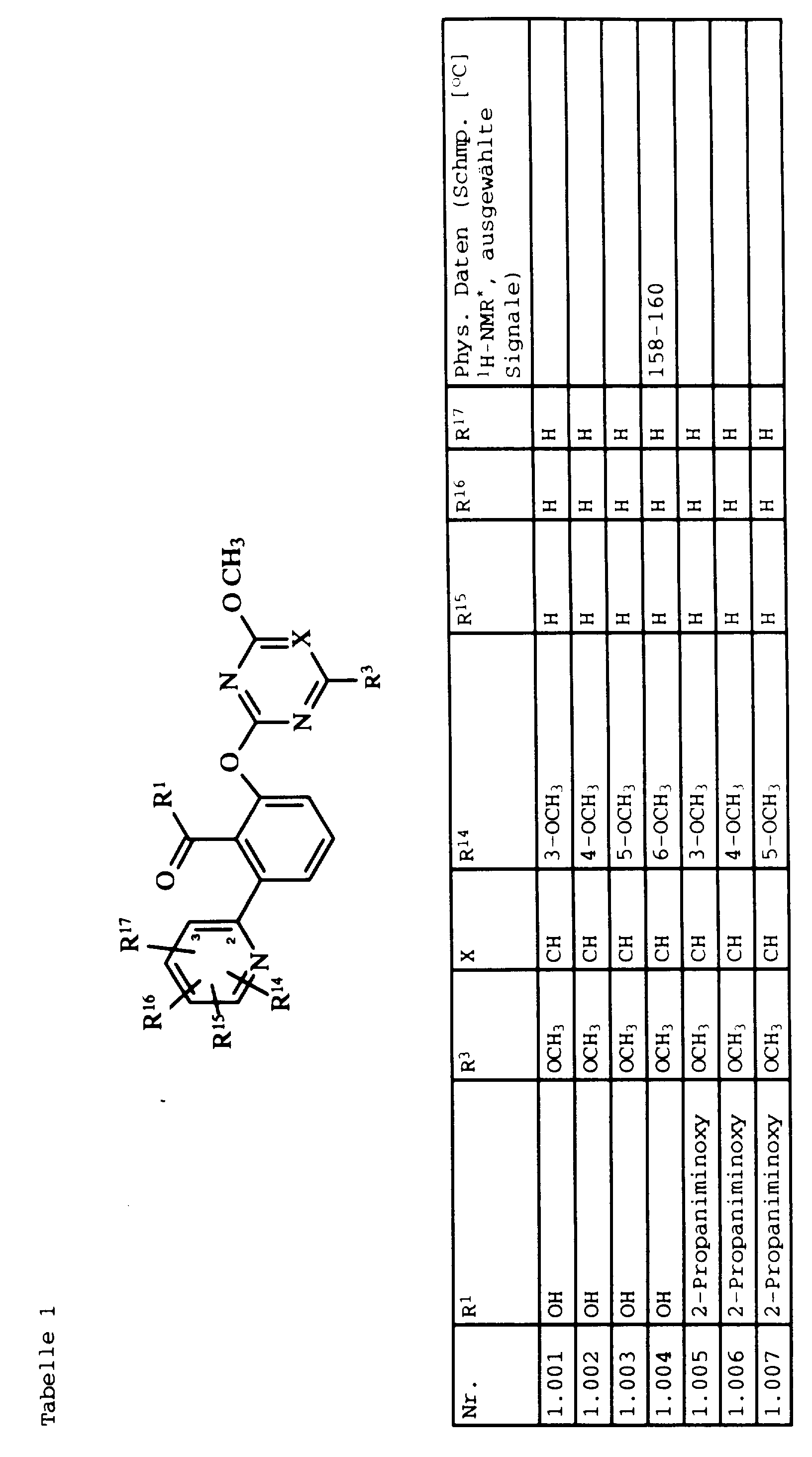

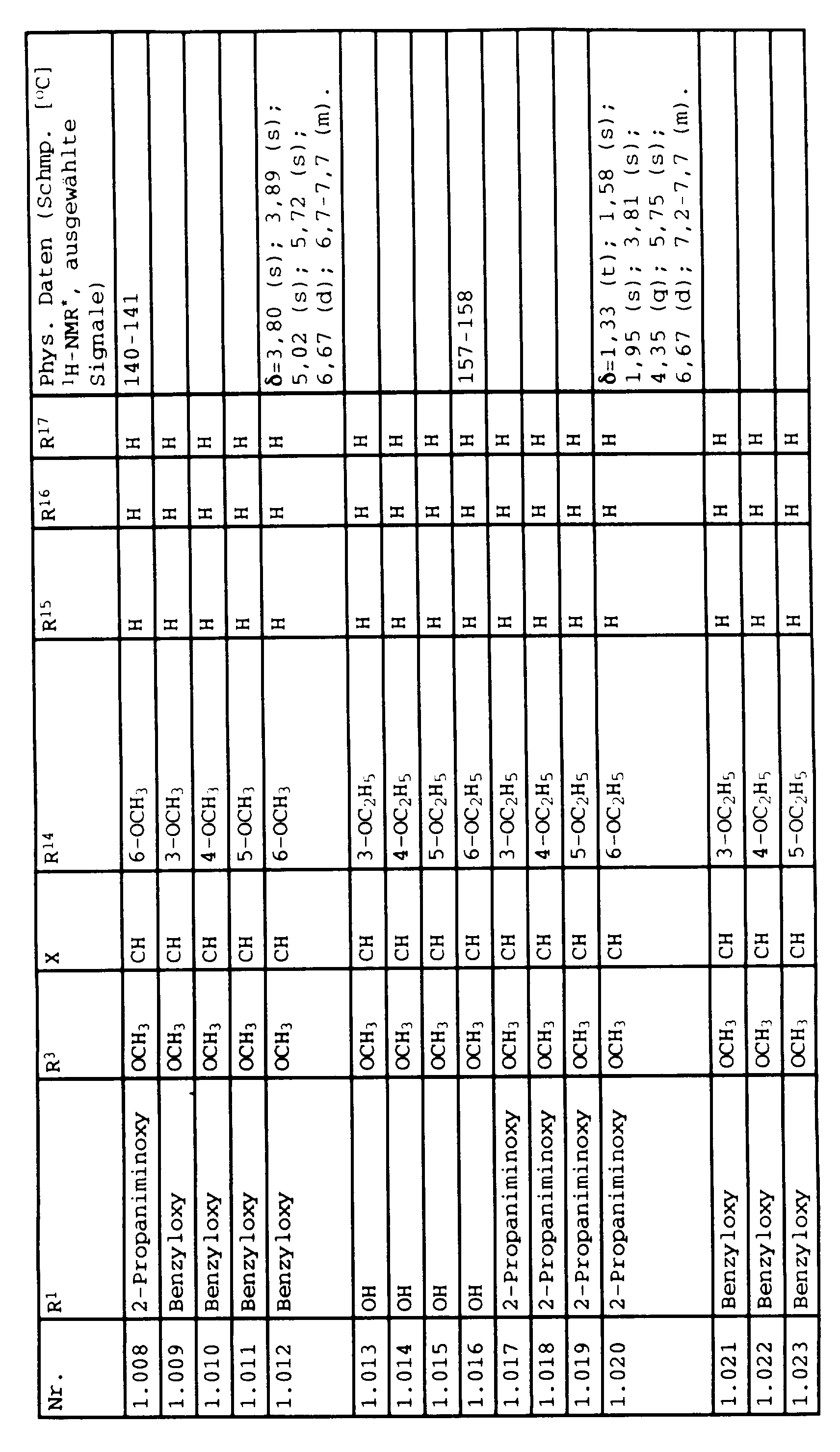

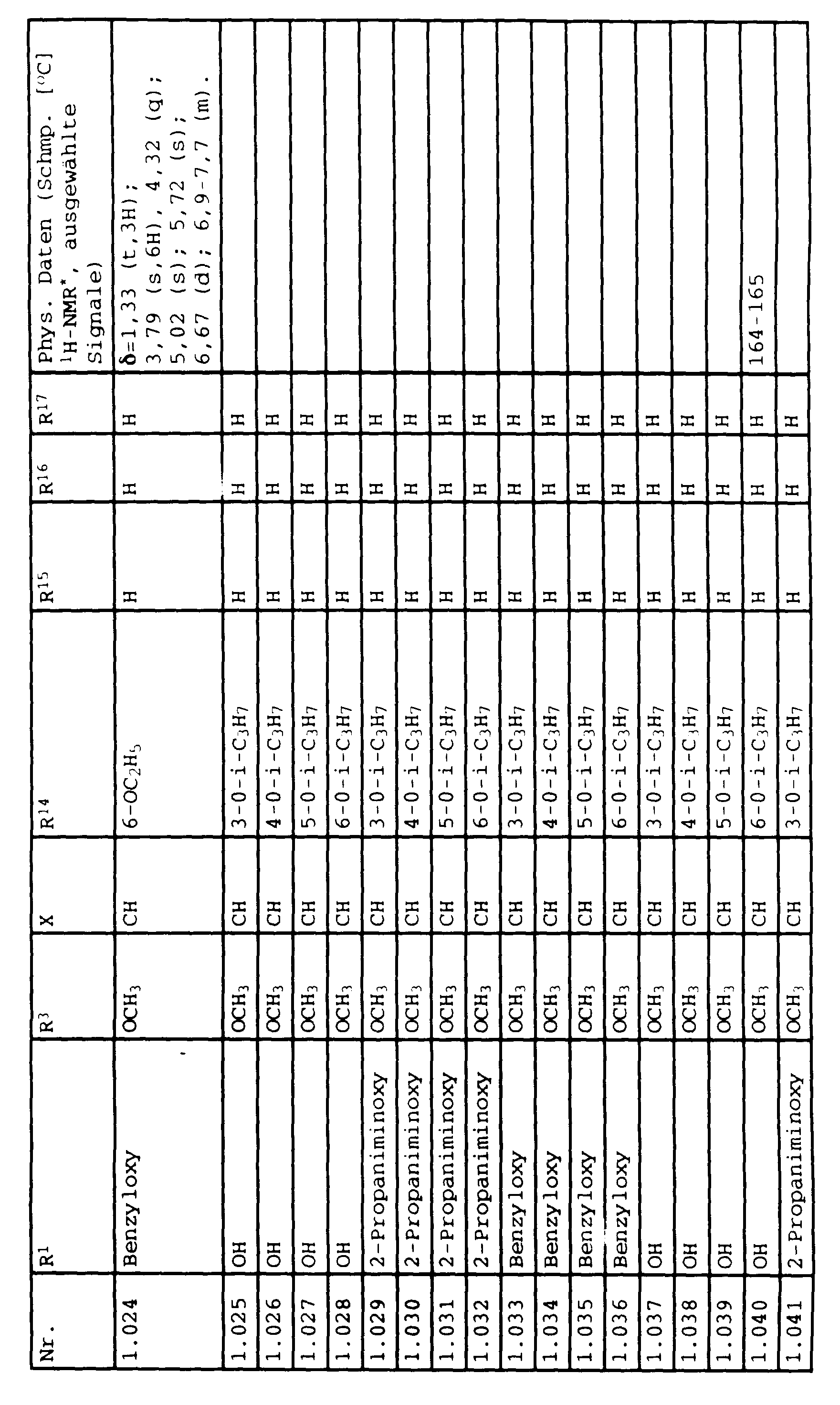

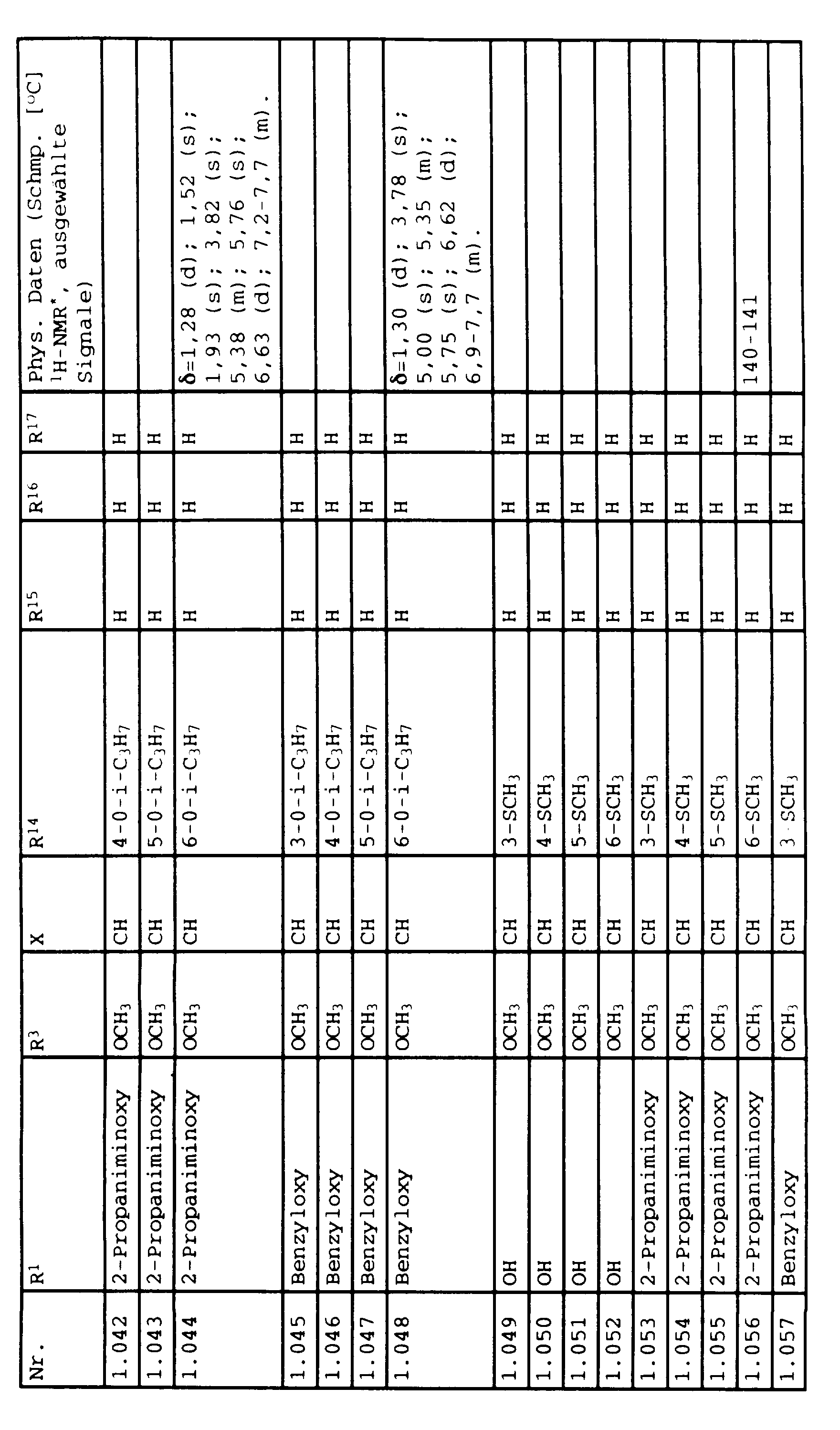

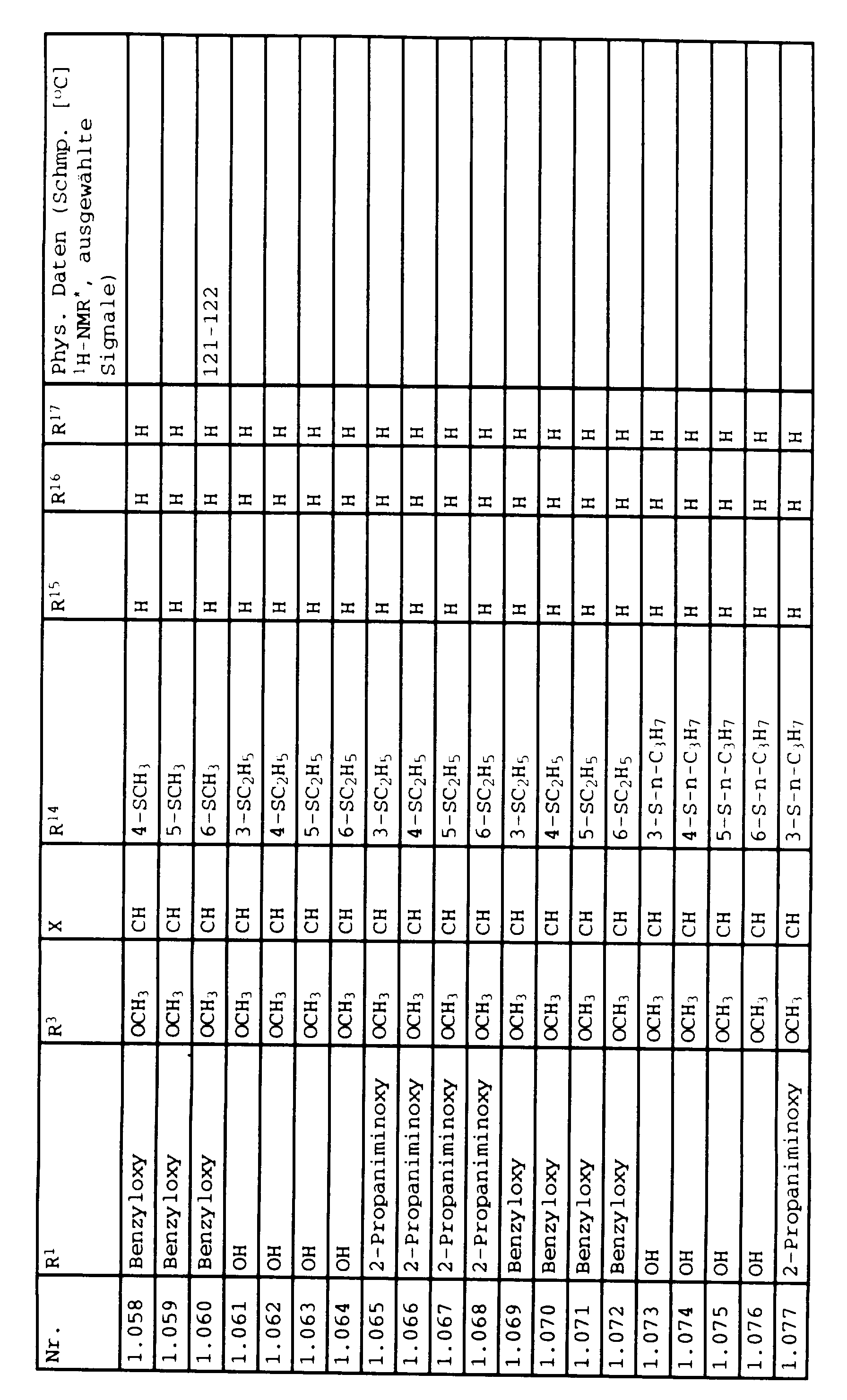

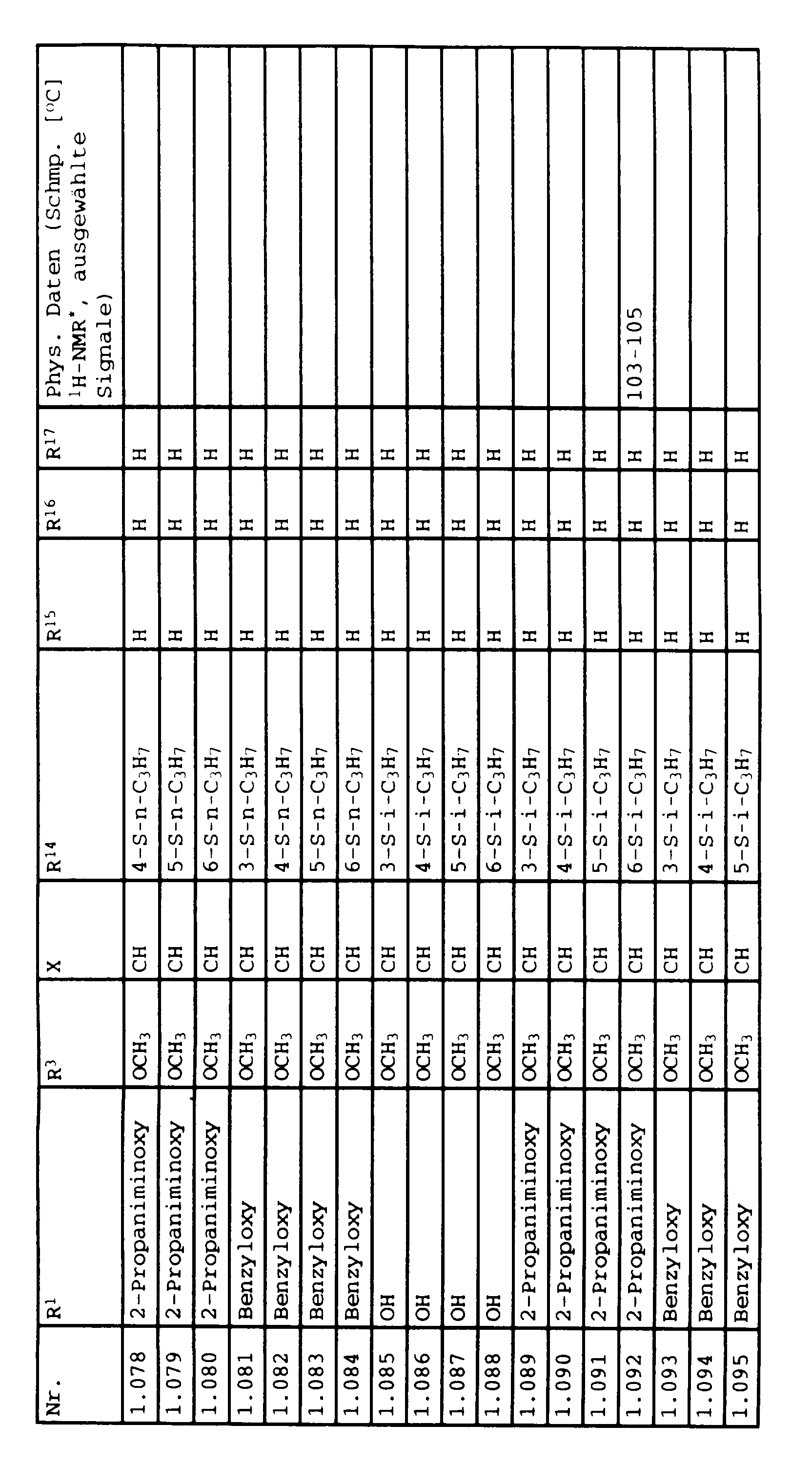

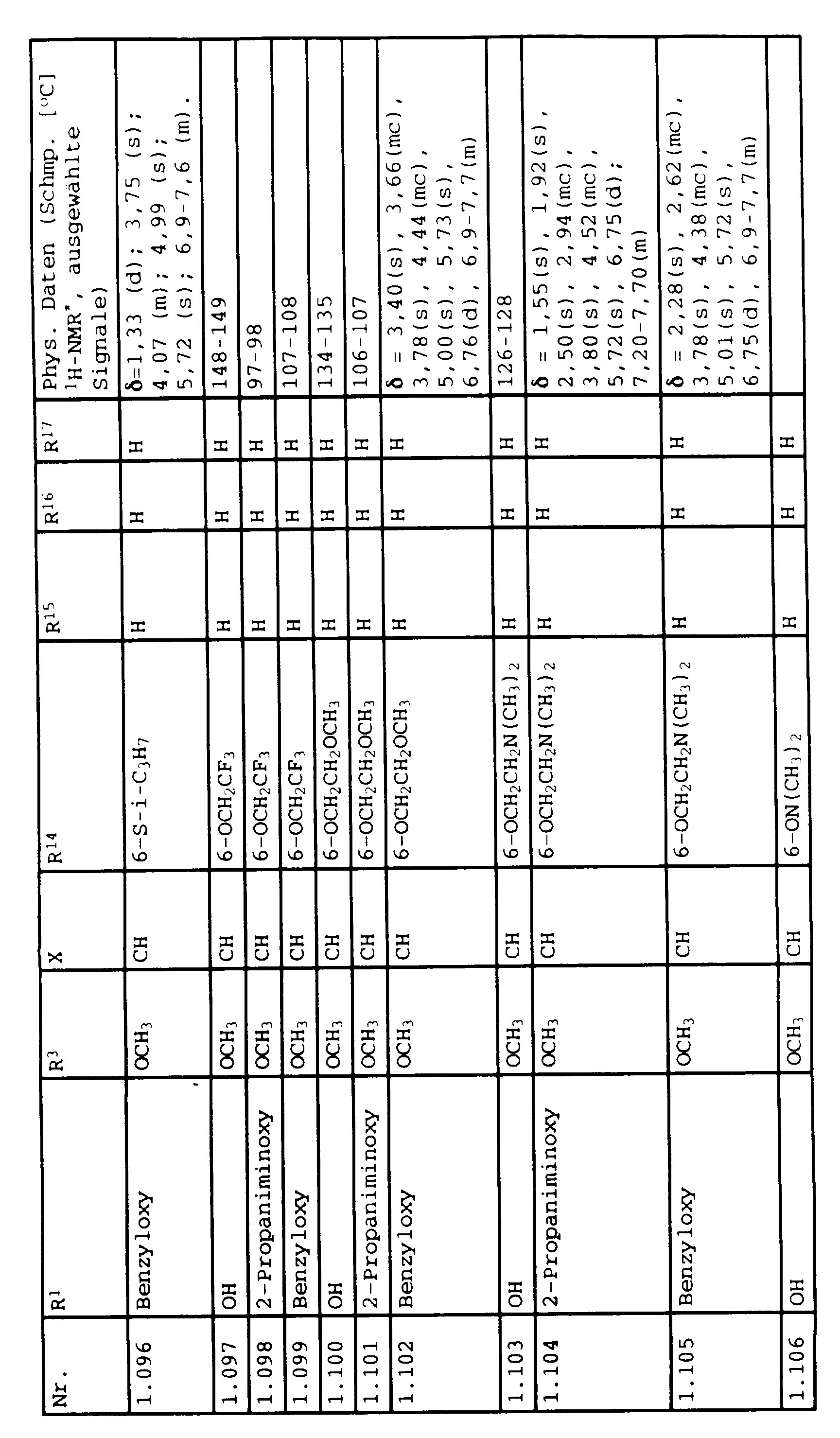

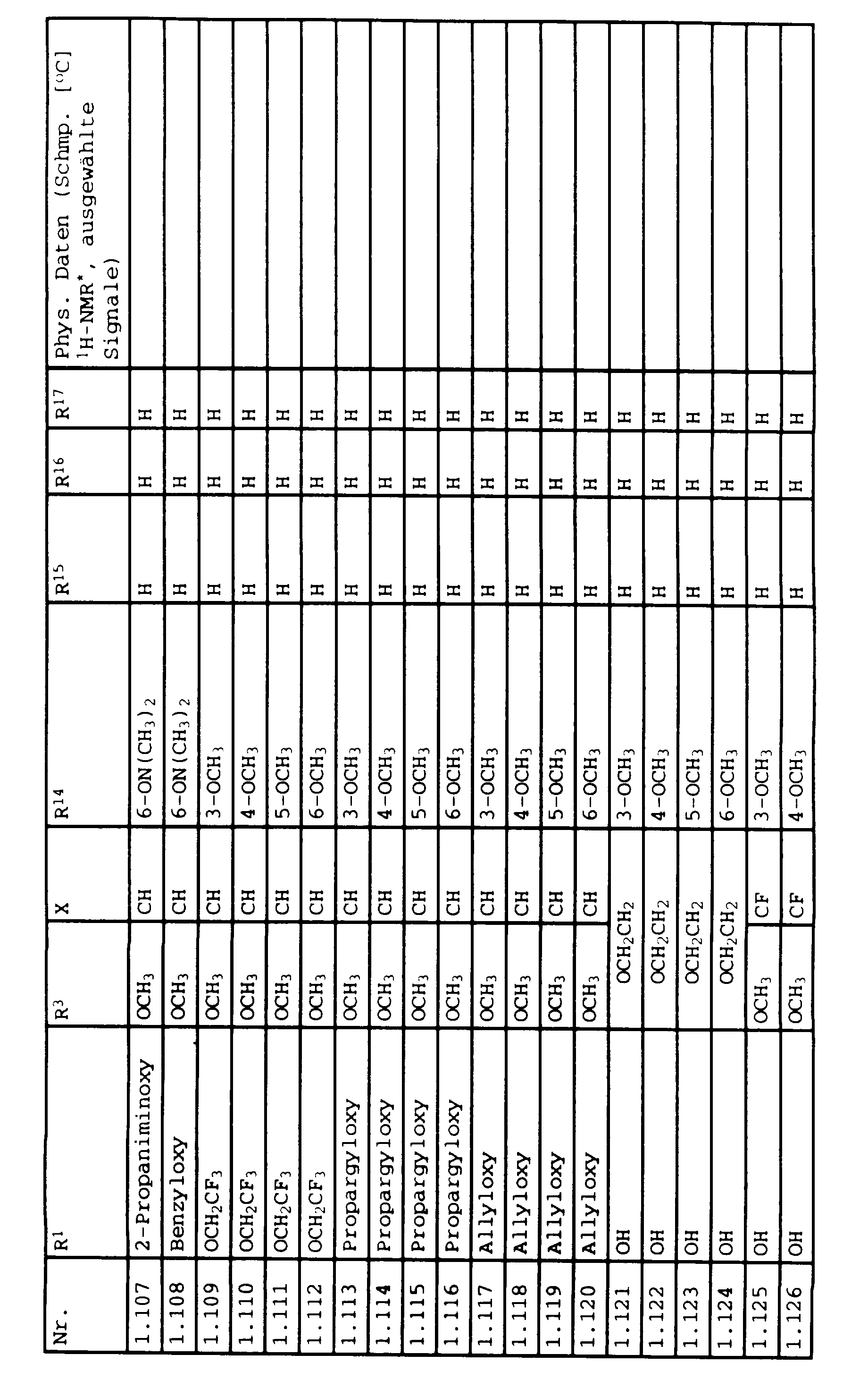

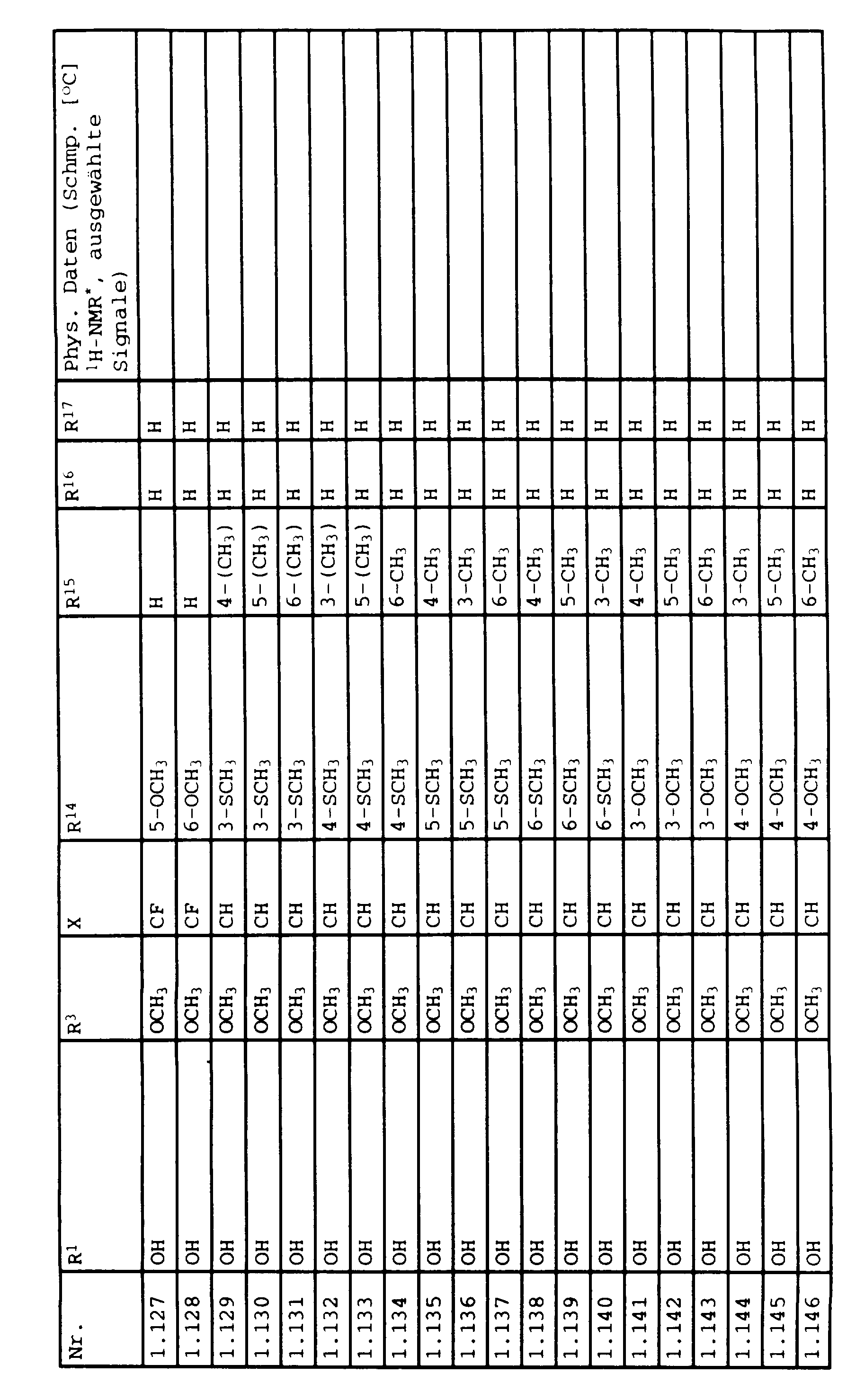

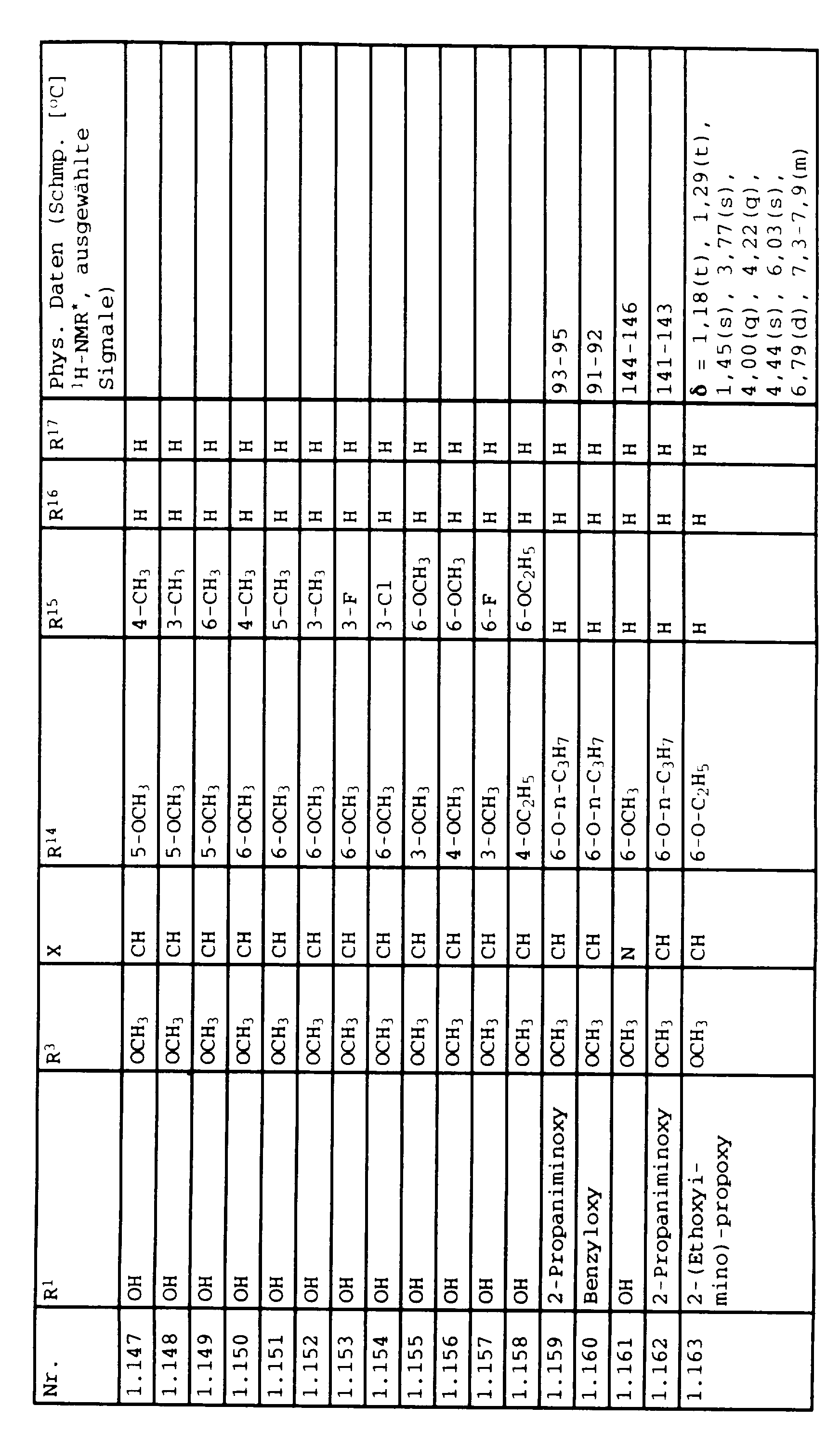

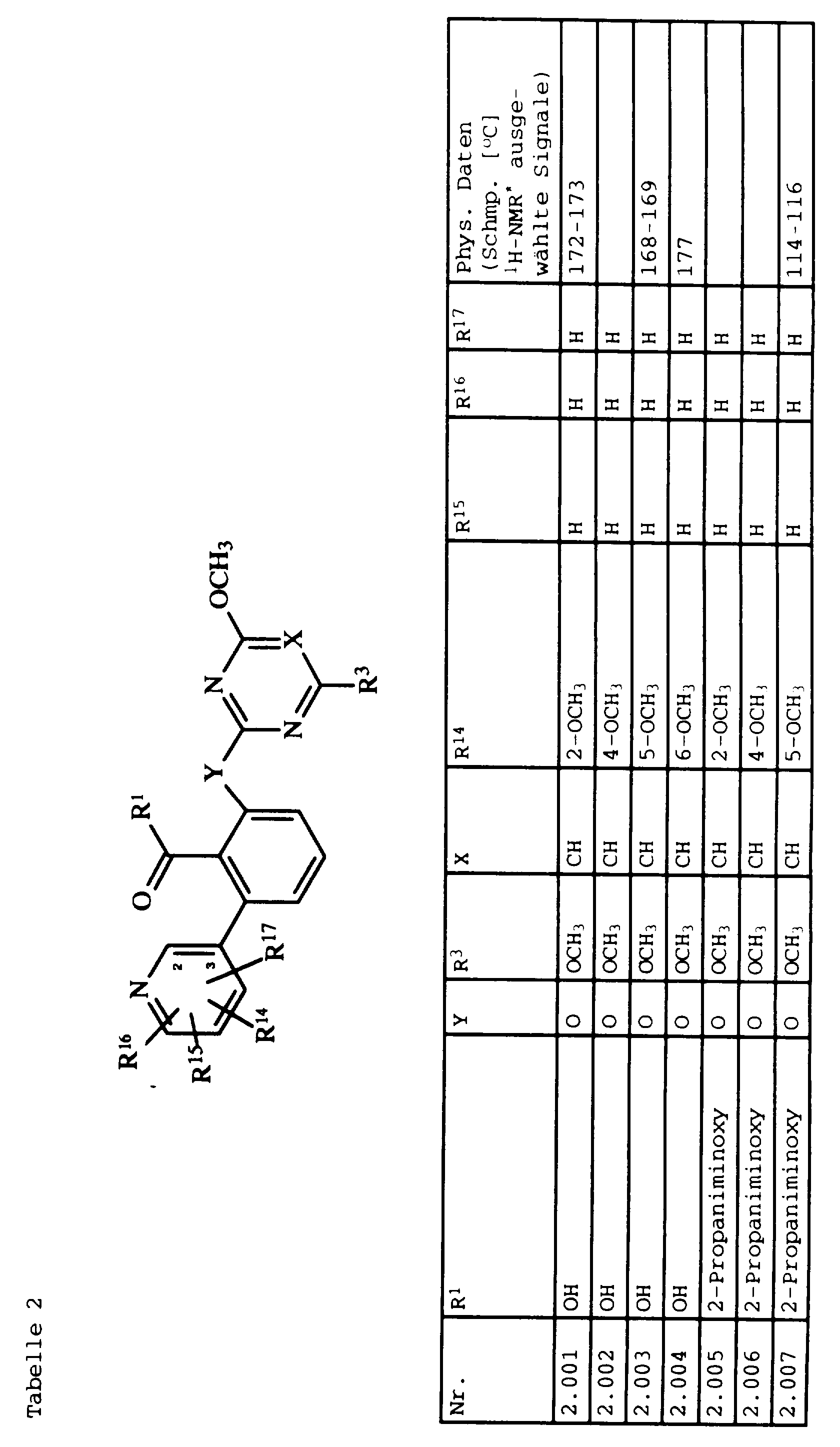

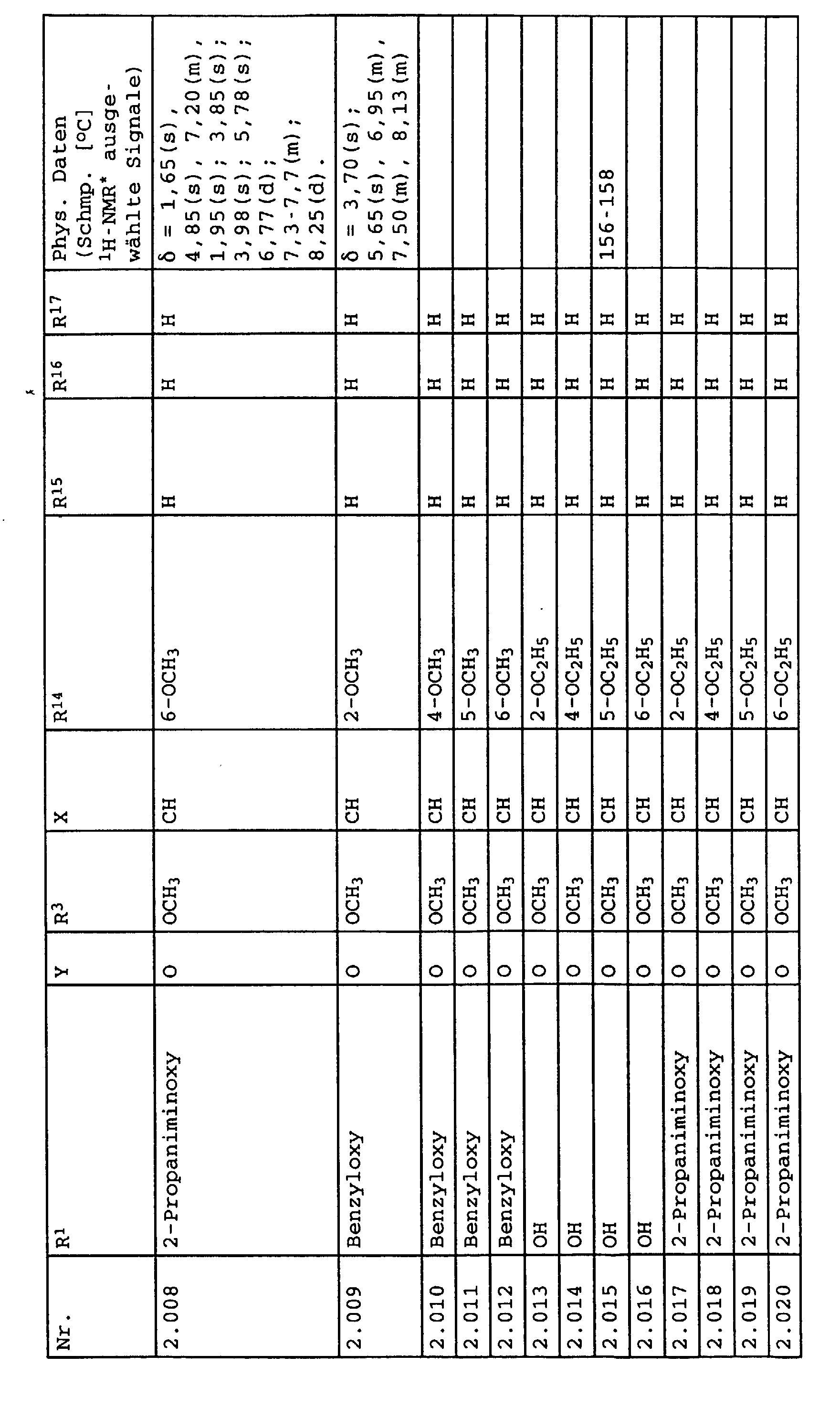

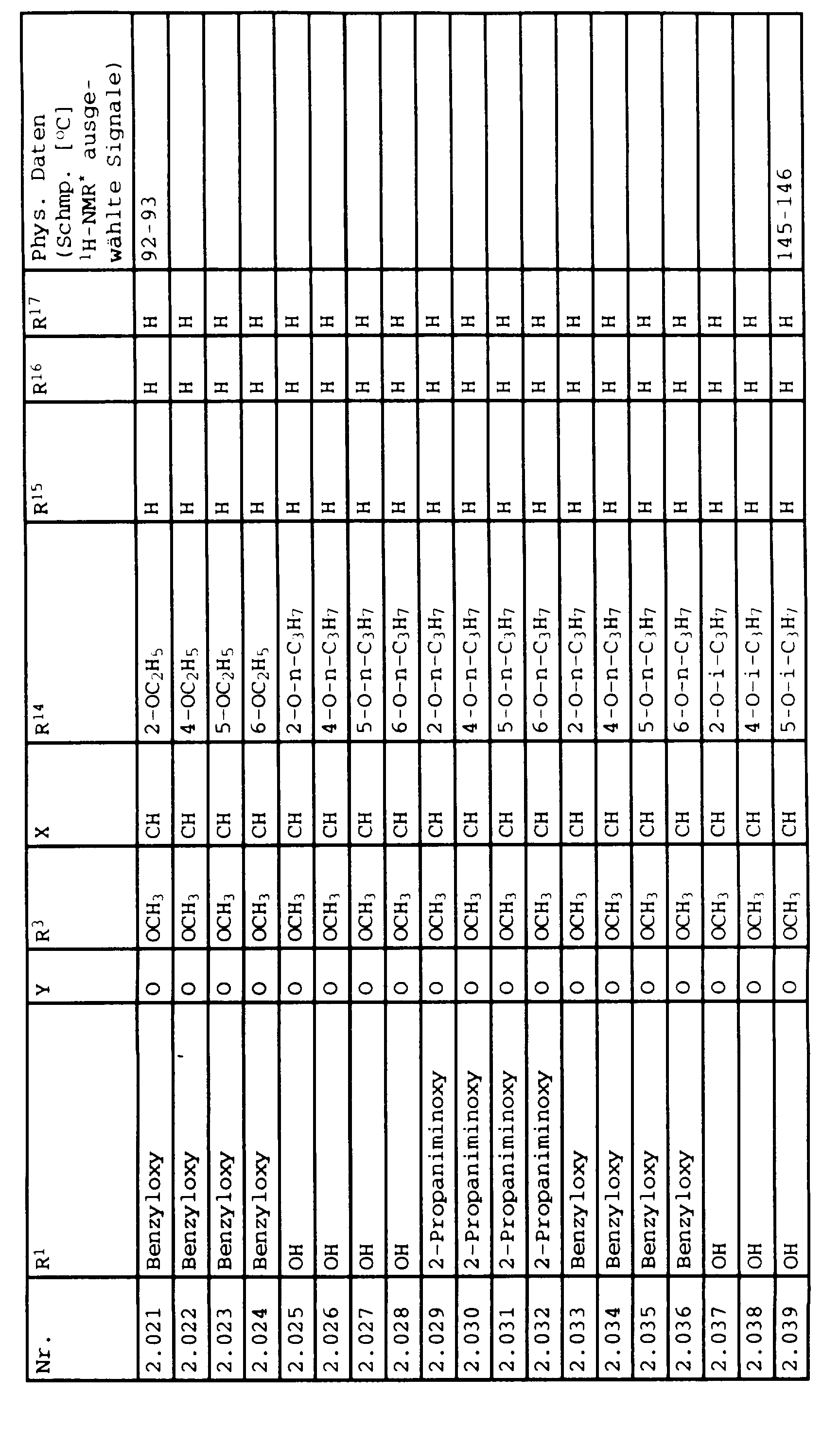

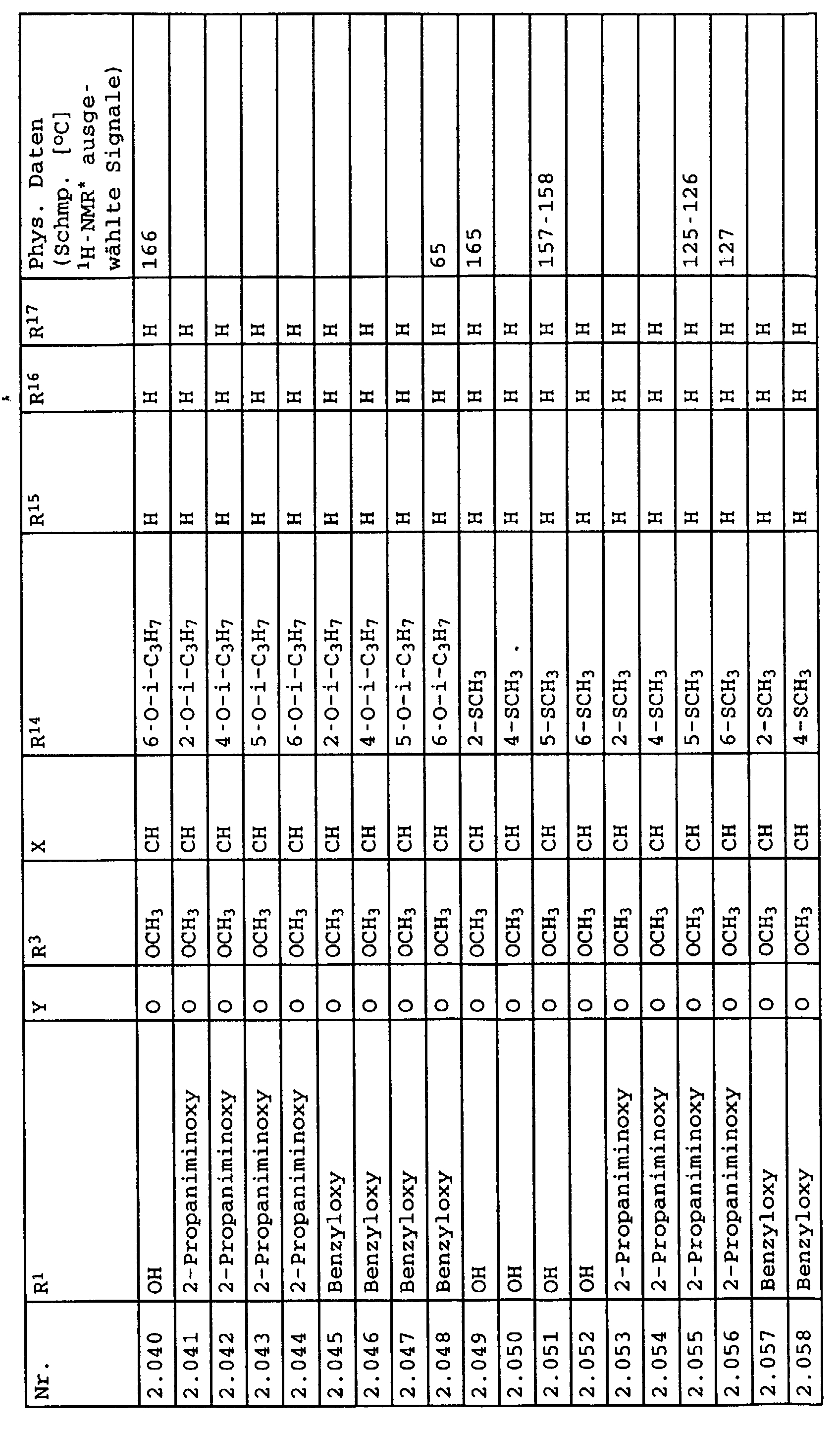

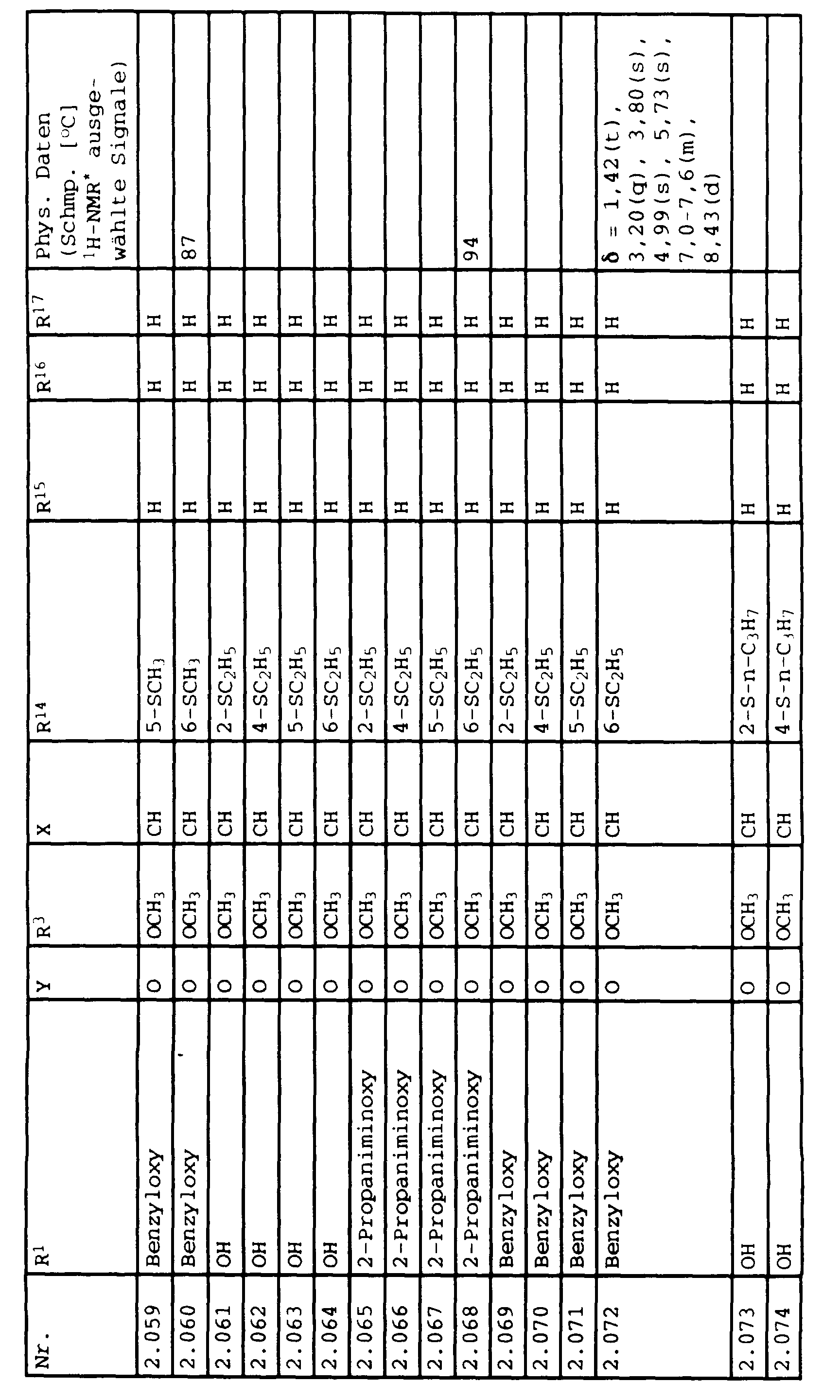

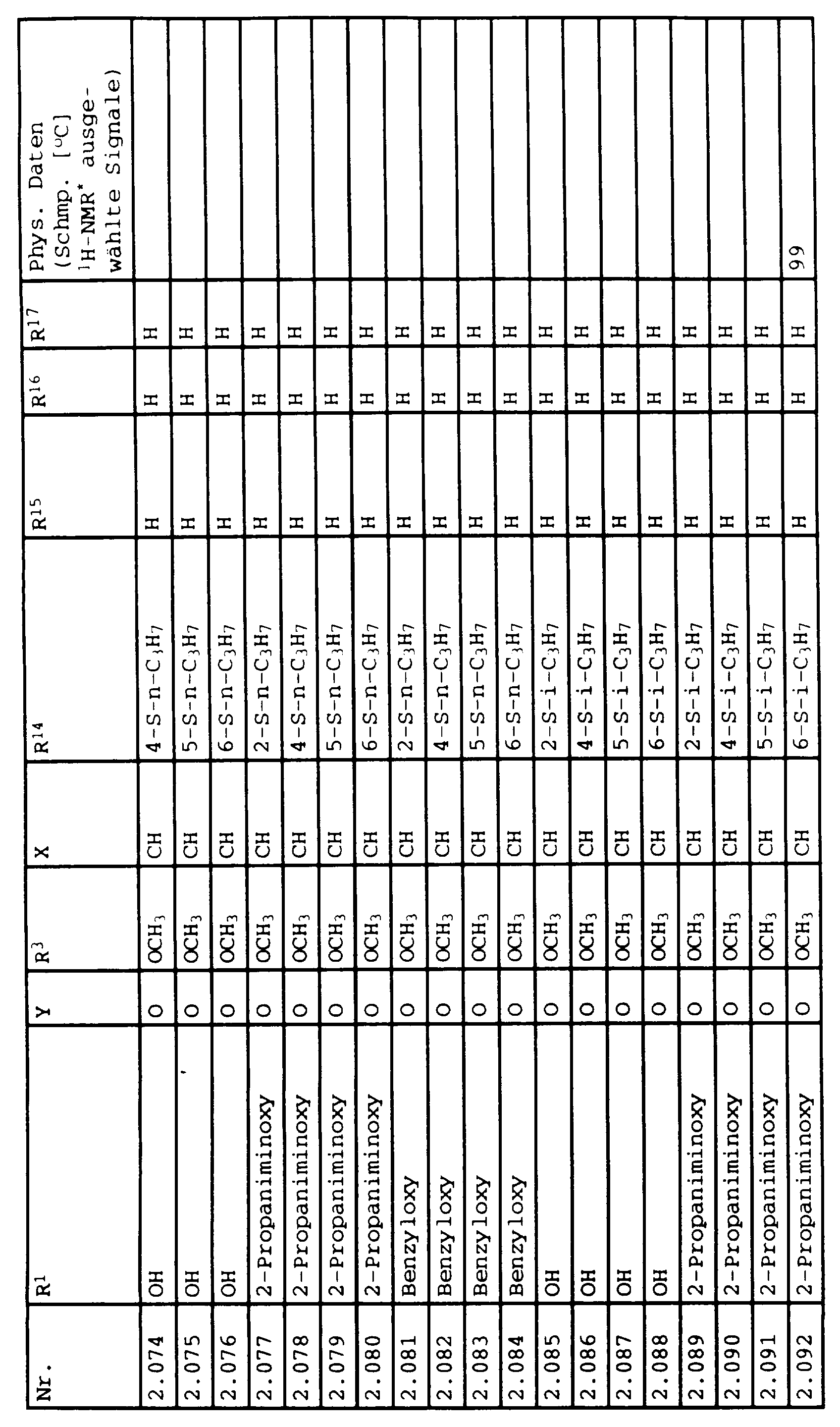

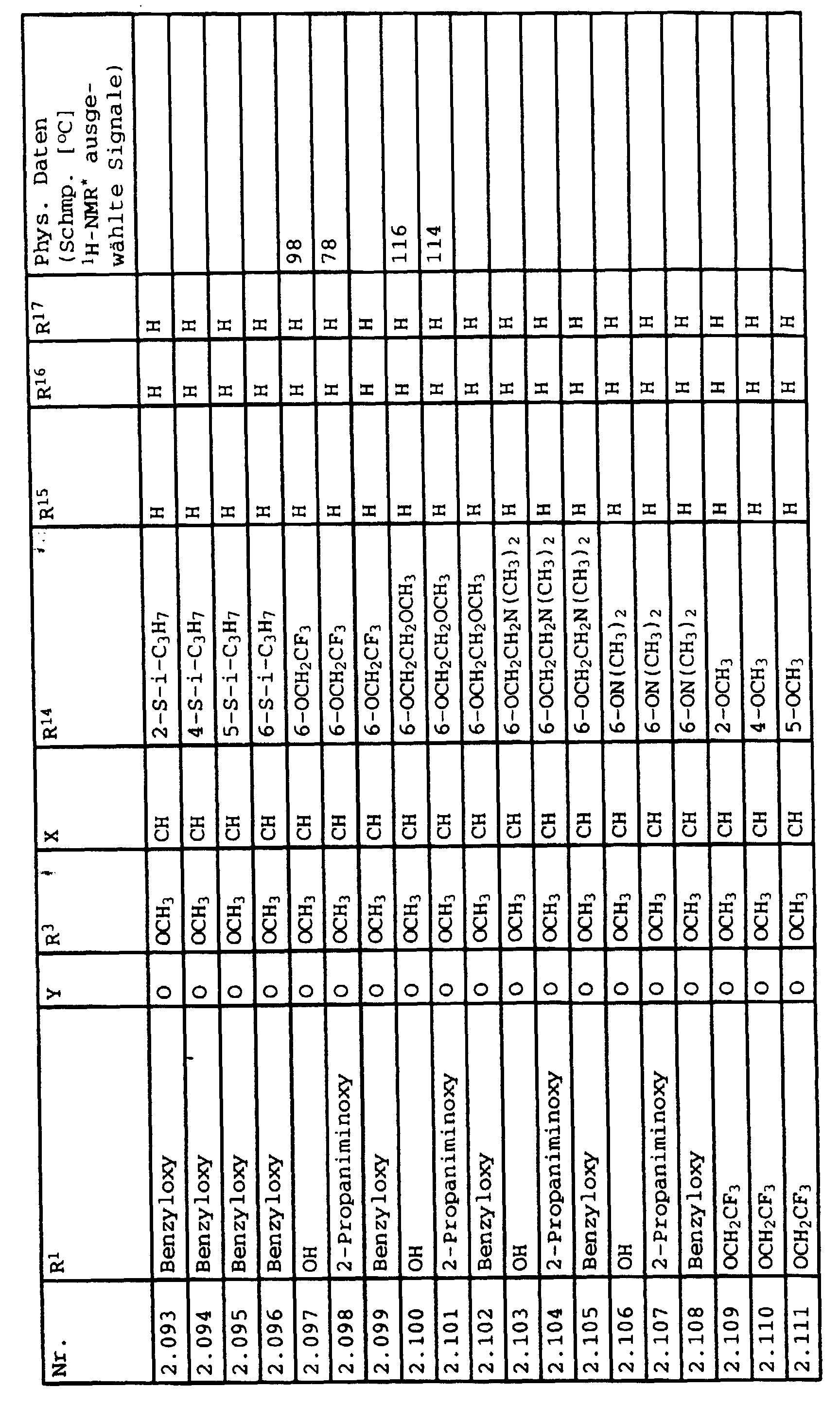

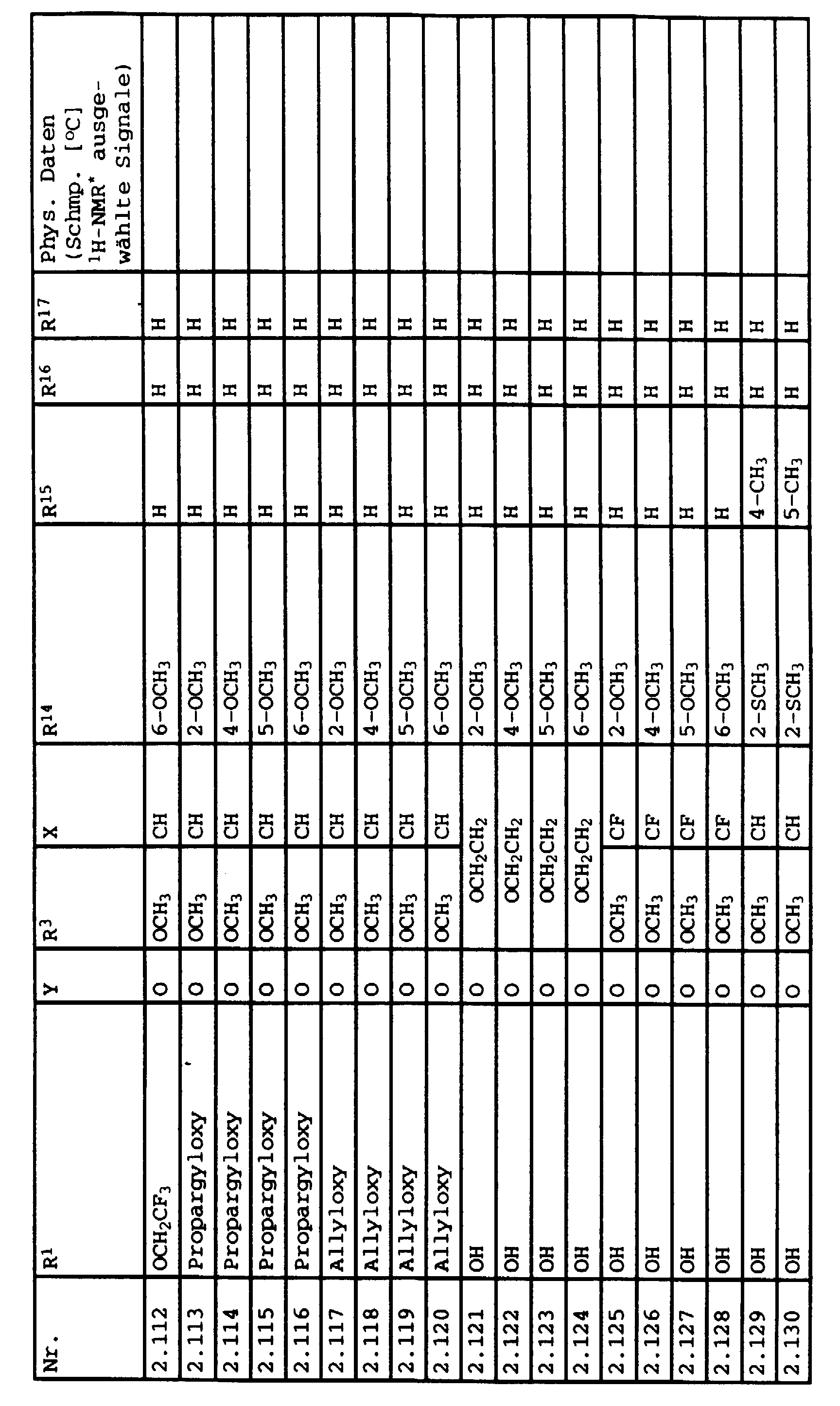

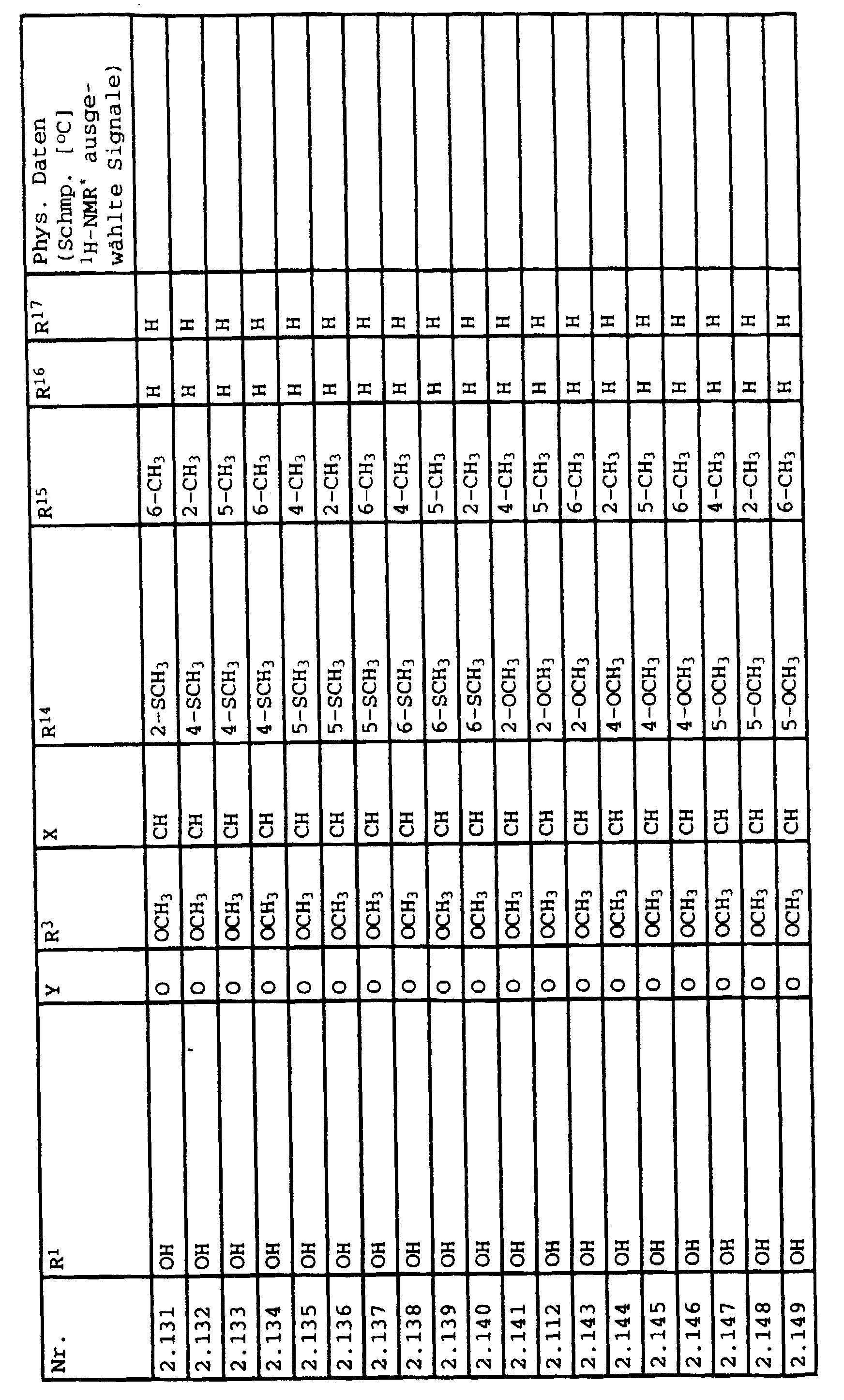

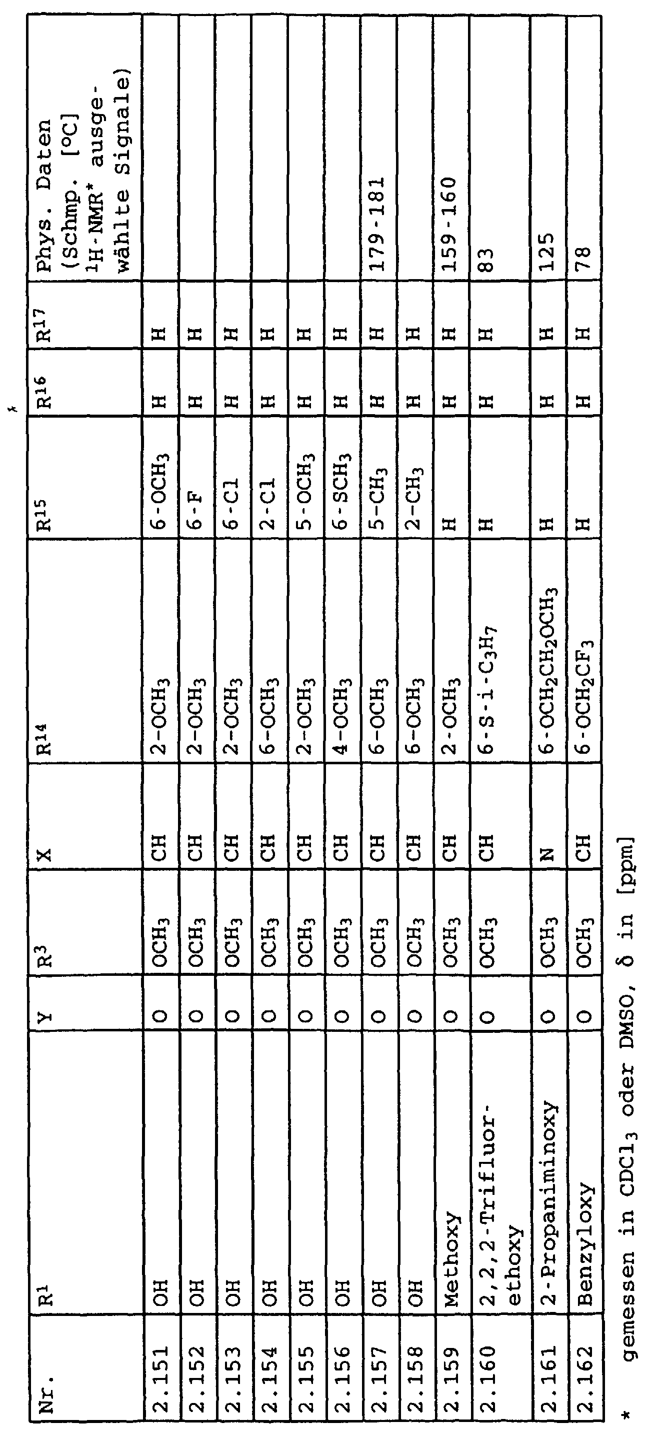

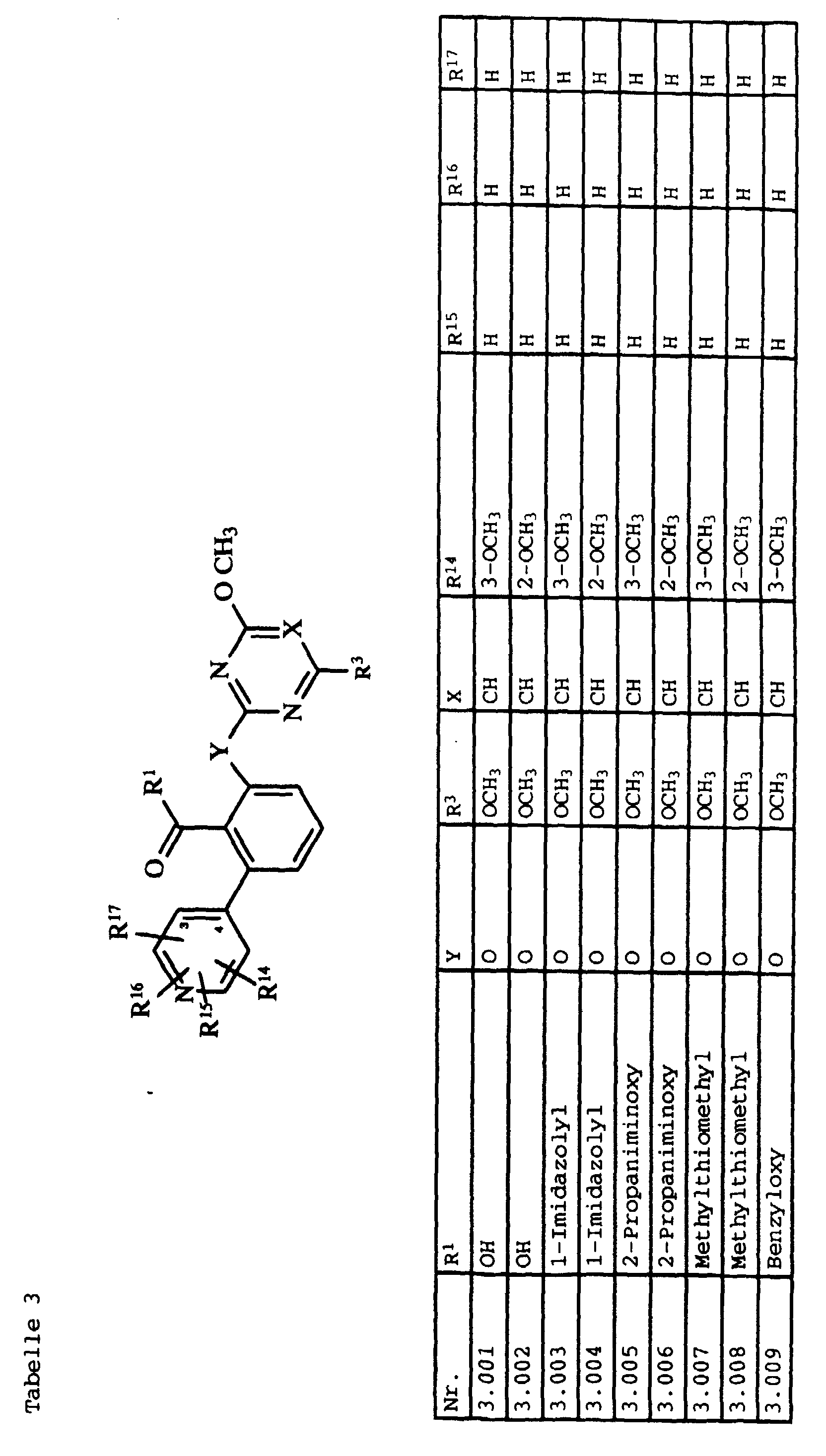

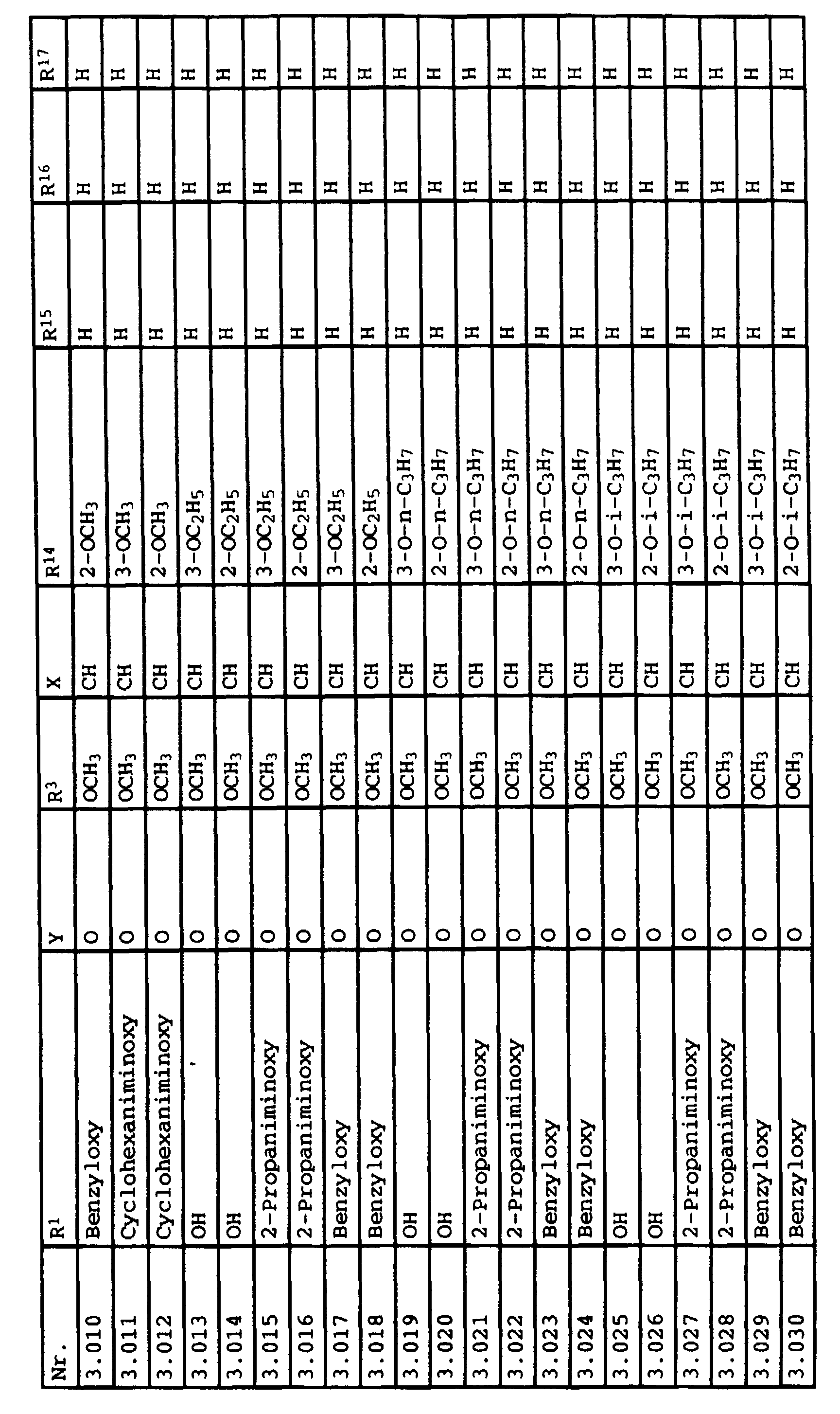

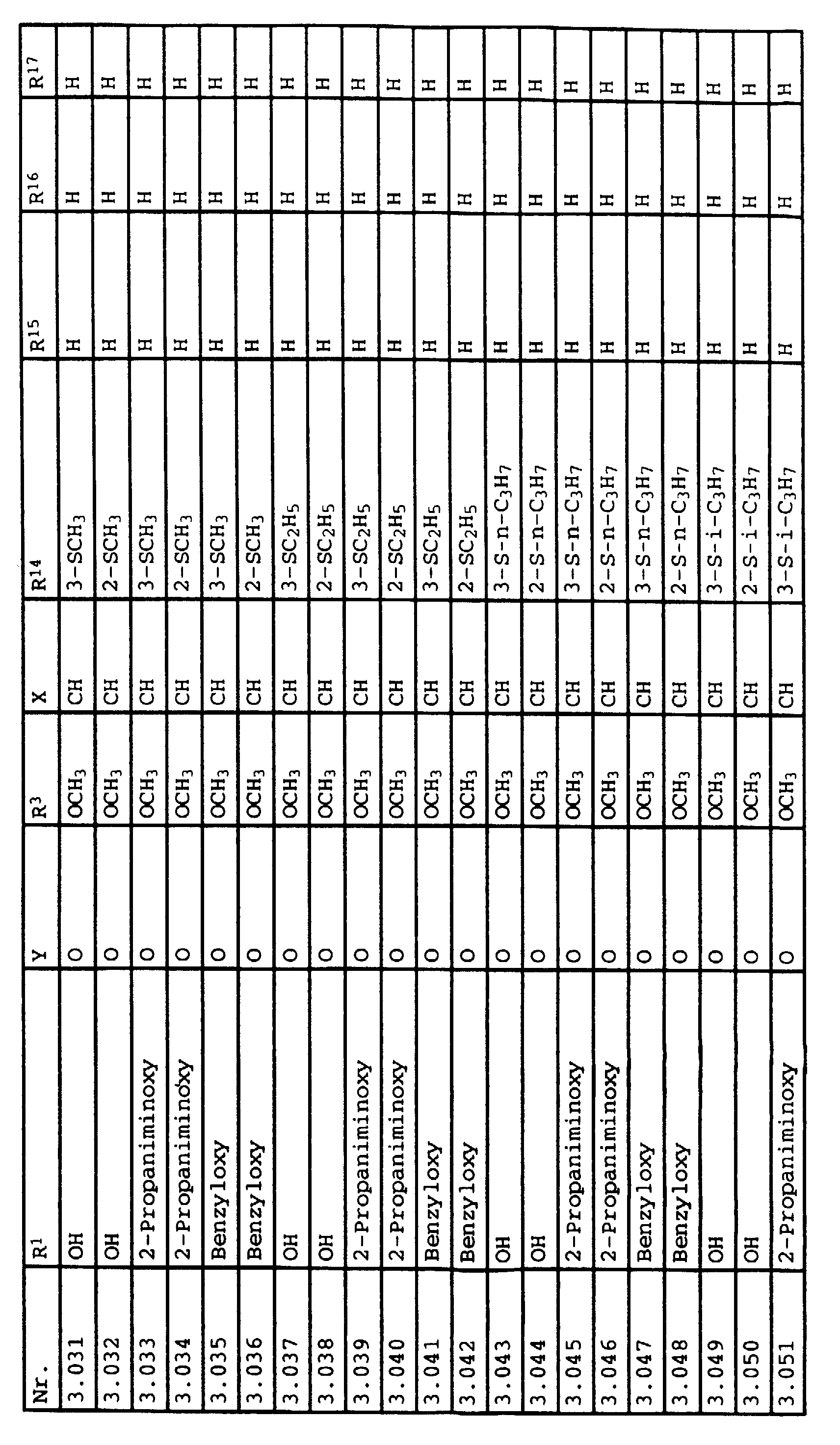

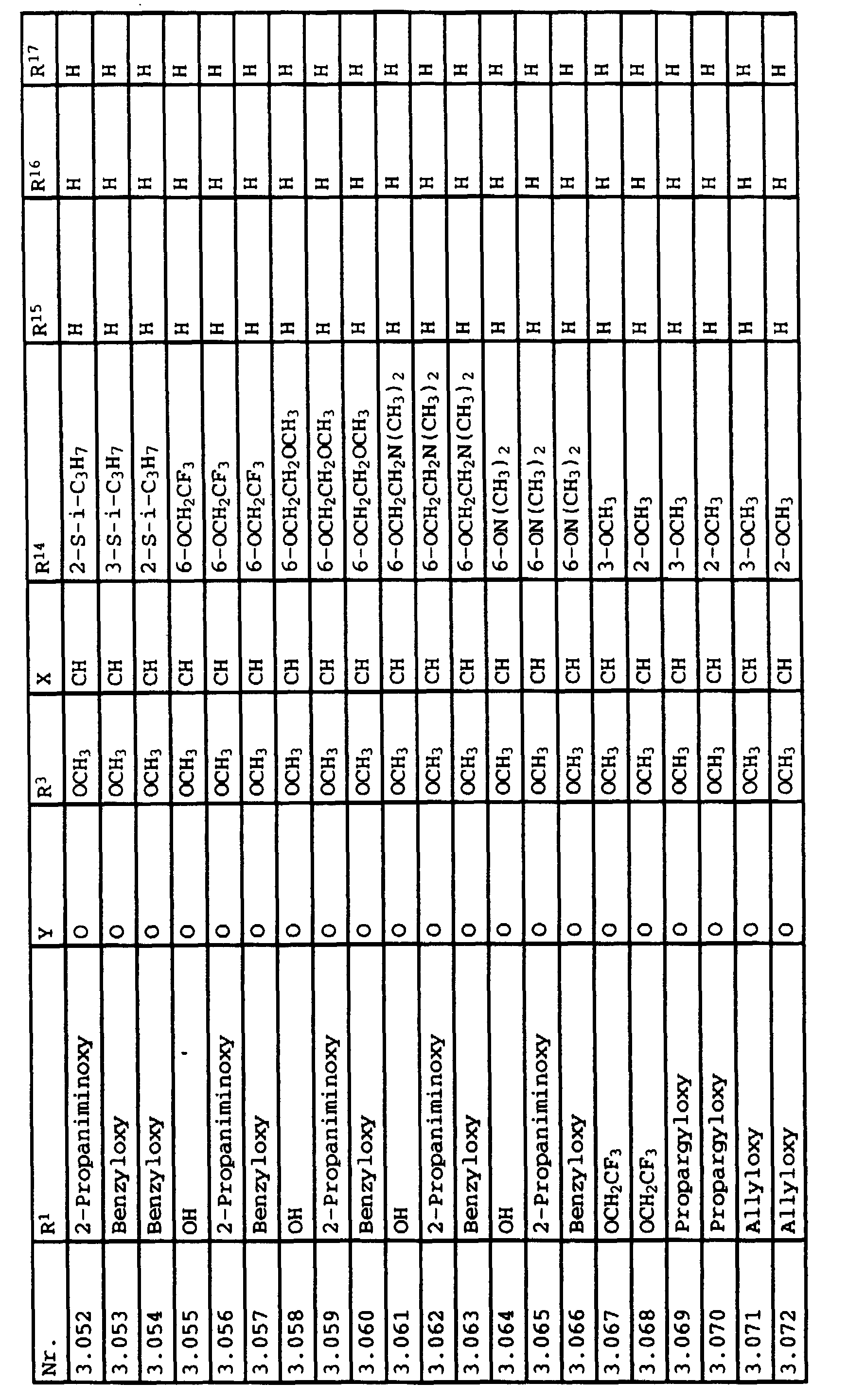

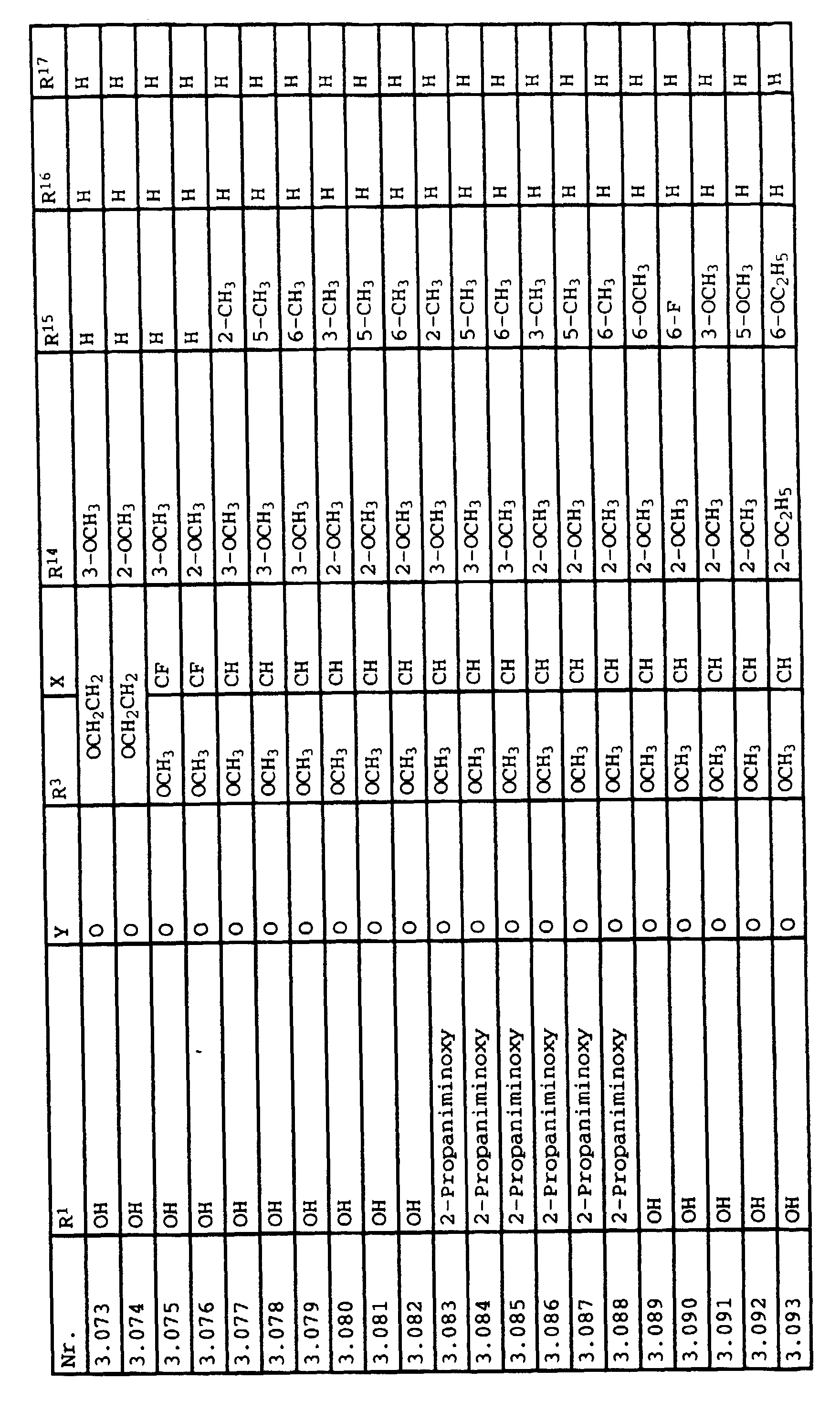

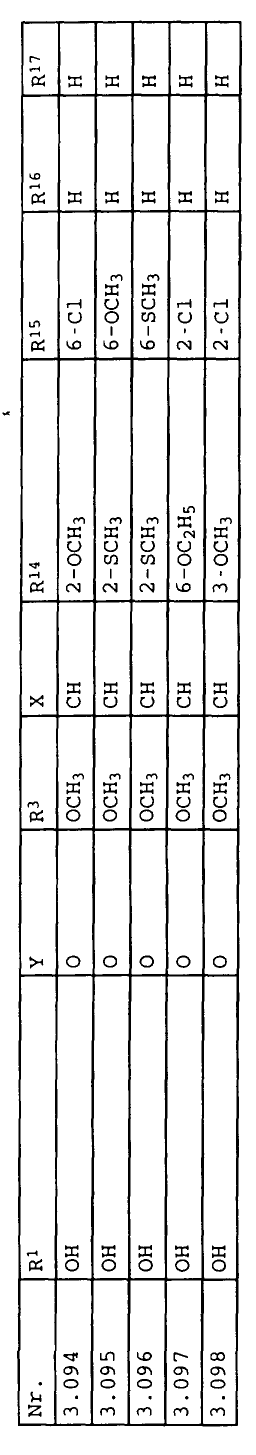

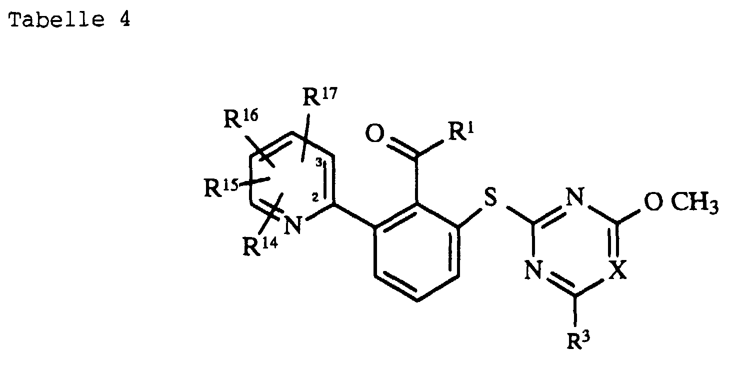

Verbindungen der o.g. Formel, in denen die Kombination der Substituenten R¹, R³, X und R¹⁴-R¹⁷ für eine Verbindung jeweils eine Zeile der Tabelle 1 entspricht. D.h. Gegenstand der Tabelle 4 sind Verbindungen der Nummern 4.001 bis 4.158, wobei Nr. 4.001 der Nr. 1.001 aus Tabelle 1, jedoch mit Y=S statt O entspricht. Verbindung Nr. 4.008 (R¹ = 2-Propaniminoxy, R³ = OCH₃, X = CH, R¹⁴ = 6-OCH₃, R¹⁵-R¹⁷ = H) weist einen Schmelzpunkt von 130-132°C auf.

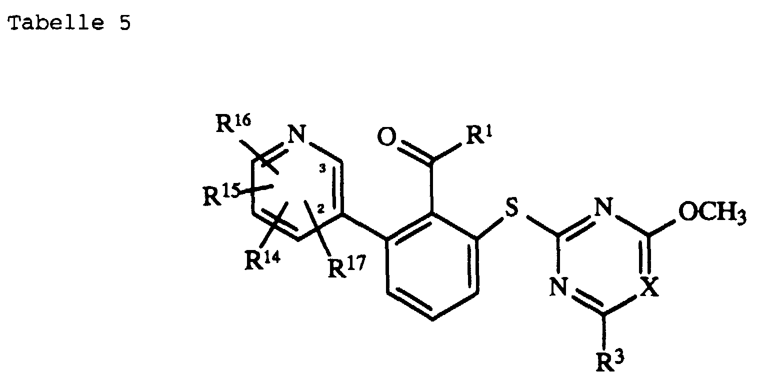

Verbindungen der o.g. Formel, in denen die Kombination der Substituenten R¹, R³, X und R¹⁴-R¹⁷ für eine Verbindung jeweils eine Zeile der Tabelle 2 entspricht. D.h. Gegenstand der Tabelle 5 sind Verbindungen der Nummern 5.001 bis 5.158, wobei Nr. 5.001 der Nr. 2.001 aus Tabelle 2, jedoch mit Y=S statt O entspricht.

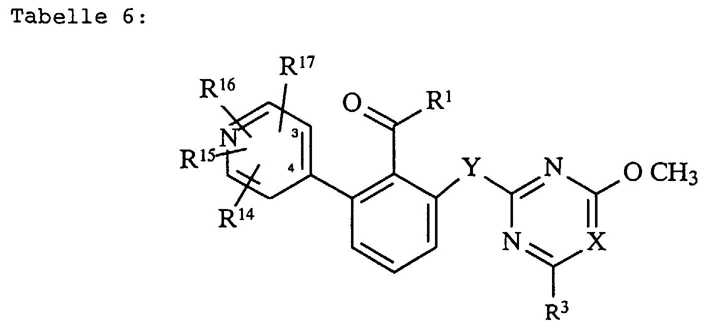

Verbindungen der o.g. Formel, in denen die Kombination der Substituenten R¹, R³, X und R¹⁴-R¹⁷ für eine Verbindung jeweils eine Zeile der Tabelle 3 entspricht. D.h. Gegenstand der Tabelle 6 sind Verbindungen der Nummern 6.001 bis 6.098, wobei Nr. 6.001 der Nr. 3.001 aus Tabelle 3, jedoch mit Y=S statt O entspricht.

### Anwendungsbeispiele

Die herbizide und wachstumsregulatorische Wirkung der Verbindungen der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,0313 bzw. 0,0625 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Die herbizide Wirkung wurde nach einer Skala von 0 bis 100 bewertet. Dabei bedeutet 100 kein Aufgang der Pflanzen bzw. völlige Zerstörung zumindest der oberirdischen Teile und 0 keine Schädigung oder normaler Wachstumsverlauf.

Die wachstumsregulierende Wirkung wurde durch Längenmessung bestimmt. Bei Versuchsende wurden die Wuchshöhen der behandelten Pflanzen gemessen und zur Wuchshöhe von nicht behandelten Pflanzen in Beziehung gesetzt.

Die in den Gewächshausversuchen verwendeten Pflanzen setzten sich aus folgenden Arten zusammen:

| Lateinischer Name | Deutscher Name | Englischer Name |
|---|---|---|
| Alopecurus myosuroides | Ackerfuchsschwanz | blackgrass |
| Echinochloa crus-galli | Hühnerhirse | barnyardgrass |
| Setaria italica | Kolbenhirse | foxtail millet |
| Sinapis alba | Weißer Senf | white mustard |
| Solanum nigrum | Schwarzer Nachtschatten | black nightshade |
| Veronica spp. | Ehrenpreisarten | speedwell |

Die Ergebnisse zeigten eine sehr gute herbizide Wirkung der erfindungsgemäßen Verbindung Nr. 1.004.

Die bessere herbizide Wirkung gegenüber Verbindungen des Standes der Technik geht aus nachstehenden Tabellen I und II hervor. Als Vergleichssubstanzen A und B dienten Verbindungen folgender Struktur:

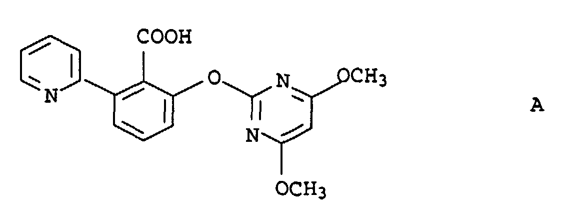

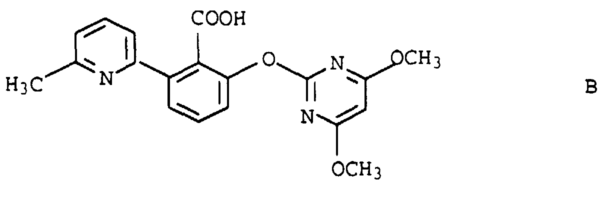

**Tabelle I:**

| Herbizide Aktivität bei Nachauflaufanwendung von 0,0625 bzw. 0,0313 kg/ha a.S. im Gewächshaus | | | | |
|---|---|---|---|---|
| Testpflanzen | Schädigung in % | | | |
| | 1.004 | | A | |
| | 0,0625 kg/ha | 0,0313 kg/ha | 0,0625 kg/ha | 0,0313 kg/ha |
| Setaria viridis | 80 | 80 | 50 | 50 |
| Sinapis alba | 100 | 100 | 90 | 85 |
| Solanum nigrum | 100 | 100 | 98 | 80 |
| Veronica spp. | 100 | 100 | 80 | 80 |

**Tabelle II:**

| Herbizide Aktivität bei Nachauflaufanwendung von 0,0156 bzw. 0,0078 kg/ha a.S. im Gewächshaus | | | | |
|---|---|---|---|---|
| Testpflanzen | Schädigung in % | | | |
| | 1.004 | | B | |
| | 0,0156 kg/ha | 0,0078 kg/ha | 0,0156 kg/ha | 0,0078 kg/ha |
| Echinochloa crus-galli | 95 | 90 | 80 | 60 |
| Sinapis alba | 98 | 98 | 95 | 90 |
| Stellaria media | 100 | 80 | 50 | 30 |
| Veronica spp. | 100 | 100 | 20 | 15 |

## Patentansprüche

1. Substituierte Pyridylsalicylaldehyd- und -Salicylsäurederivate der allgemeinen Formel I, 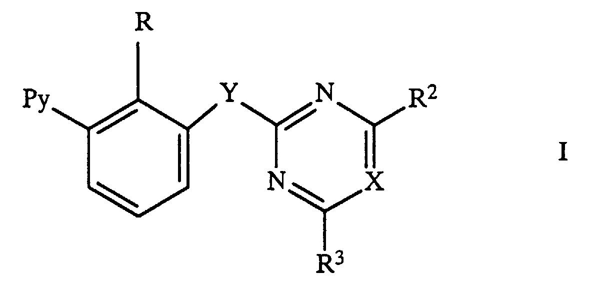 in der R eine Formylgruppe, eine Gruppe CO₂H oder einen zu CO₂H hydrolysierbaren Rest bedeutet und die übrigen Substituenten folgende Bedeutung haben:
R² Halogen, C₁-C₄-Alkyl, C₁-C₄-Halogenalkyl, C₁-C₄-Alkoxy, C₁-C₄-Halogenalkoxy oder C₁-C₄-Alkylthio;
X Stickstoff oder CR¹³, wobei R¹³ Wasserstoff oder Halogen bedeutet oder zusammen mit R³ eine 3- bis 4-gliedrige Alkylen- oder Alkenylenkette bildet, in der jeweils eine Methylengruppe durch Sauerstoff ersetzt ist;
R³ Halogen, C₁-C₄-Alkyl, C₁-C₄-Halogenalkyl, C₁-C₄-Alkoxy, C₁-C₄-Halogenalkoxy, C₁-C₄-Alkylthio, oder R³ ist mit R¹³ wie oben angegeben zu einem 5- oder 6-gliedrigen Ring verknüpft;
Y Sauerstoff oder Schwefel;
Py ein in beliebiger Position verknüpfter Pyridinring, der vier Substituenten R¹⁴, R¹⁵, R¹⁶ und R¹⁷ trägt;
R¹⁴
a) eine C₁-C₈-Alkoxygruppe, welche ein bis fünf Halogenatome und/oder einen der folgenden Reste tragen kann:
C₁-C₄-Alkoxy, C₁-C₄-Halogenalkoxy, C₁-C₄-Alkylthio, Cyano, C₃-C₈-Cycloalkyl oder Di-C₁-C₄-Alkylamino;
b) eine C₁-C₄-Alkylthiogruppe, welche ein bis fünf Halogenatome und/oder einen der folgenden Reste tragen kann:
C₁-C₄-Alkoxy, C₁-C₄-Halogenalkoxy, C₁-C₄-Alkylthio, Cyano, C₃-C₈-Cycloalkyl oder Di-C₁-C₄-Alkylamino;
R¹⁵, R¹⁶, R¹⁷ jeweils unabhängig voneinander Wasserstoff, Nitro, Halogen, C₁-C₈-Alkyl, C₁-C₈-Halogenalkyl sowie die bei R¹⁴ genannten Reste.

2. Substituierte Pyridylsalicylaldehyd- und -Salicylsäurederivate der Formel I gemäß Anspruch 1, in der R für eine Gruppe 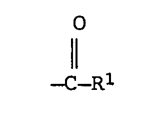 steht, wobei R¹ die folgende Bedeutung hat:
a) Wasserstoff;
b) eine Succinylimidoxygruppe;
c) ein über ein Stickstoffatom verknüpfter 5-gliedriger Heteroaromat, enthaltend zwei bis drei Stickstoffatome, welcher ein bis zwei Halogenatome und/oder ein bis zwei der folgenden Reste tragen kann:
C₁-C₄-Alkyl, C₁-C₄-Halogenalkyl, C₁-C₄-Alkoxy, C₁-C₄-Halogenalkoxy und/oder C₁-C₄-Alkylthio;
d) ein Rest -(O)ₘ-NR⁶R⁷, in dem m für 0 oder 1 steht und R⁶ und R⁷, die gleich oder unterschiedlich sein können, die folgende Bedeutung haben:
Wasserstoff;
C₁-C₈-Alkyl, C₃-C₆-Alkenyl, C₃-C₆-Alkinyl, C₃-C₈-Cycloalkyl, wobei diese Reste jeweils ein bis fünf Halogenatome und/oder ein bis zwei der folgenden Gruppen tragen können: C₁-C₄-Alkyl, C₁-C₄-Alkoxy, C₃-C₆-Alkenyloxy, C₃-C₆-Alkinyloxy, C₁-C₄-Alkylthio, C₃-C₆-Alkenylthio, C₃-C₆-Alkinylthio, C₁-C₄-Halogenalkoxy, C₁-C₄-Alkylcarbonyl, C₃-C₆-Alkenylcarbonyl, C₃-C₆-Alkinylcarbonyl, C₁-C₄-Alkoxycarbonyl, C₃-C₆-Alkenyloxycarbonyl,
C₃-C₆-Alkinyloxycarbonyl, Di-(C₁-C₄-alkyl)amino, C₃-C₈-Cycloalkyl, Phenyl, ein oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl;
Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
R⁶ und R⁷ gemeinsam eine zu einem Ring geschlossene, optionell substituierte C₄-C₇-Alkylenkette oder gemeinsam eine zu einem Ring geschlossene, optionell substituierte C₃-C₆-Alkylenkette mit einem Heteroatom, ausgewählt aus der Gruppe Sauerstoff, Schwefel oder Stickstoff;
e) R¹ ferner eine Gruppe 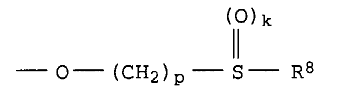 in der R⁸ für C₁-C₄-Alkyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl, C₁-C₄-Halogenalkyl, C₃-C₆-Alkenyl oder C₃-C₆-Alkinyl steht, p die Werte 1, 2, 3 oder 4 und k die Werte 0, 1 oder 2 annehmen;
f) einen Rest OR⁹, worin R⁹ bedeutet:
i) Wasserstoff, ein Alkalimetallkation, das Äquivalent eines Erdalkalimetallkations, das Ammoniumkation oder ein organisches Ammoniumion;
ii) eine C₃-C₈-Cycloalkylgruppe, welche ein bis drei C₁-C₄-Alkylreste tragen kann;
iii) eine C₁-C₈-Alkylgruppe, welche ein bis fünf Halogenatome und/oder einen der folgenden Reste tragen kann:
C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio substituiertes Phenyl oder Phenoxy;
iv) eine C₁-C₈-Alkylgruppe, welche ein bis fünf Halogenatome tragen kann und einen der folgenden Reste trägt: ein 5-gliedriger Heteroaromat, enthaltend ein bis drei Stickstoffatome, oder ein 5-gliedriger Heteroaromat, enthaltend ein Stickstoffatom und ein Sauerstoff- oder Schwefelatom, welche ein bis vier Halogenatome und/oder ein bis zwei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
v) eine C₂-C₆-Alkylgruppe, welche in der 2-Position einen der folgenden Reste trägt: C₁-C₄-Alkoxyimino, C₃-C₆-Alkenyloxyimino, C₃-C₆-Halogenalkenyloxyimino oder Benzyloxyimino;
vi) eine C₃-C₆-Alkenyl- oder eine C₃-C₆-Alkinylgruppe, wobei diese Gruppen ihrerseits ein bis fünf Halogenatome tragen können;
vii) ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
viii)ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
ix) R⁹ ferner eine Gruppe -N=CR¹⁰R¹¹, worin R¹⁰ und R¹¹, die gleich oder verschieden sein können, bedeuten:
C₁-C₁₂-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio- und/oder einen Phenylrest tragen können;
Phenyl, das durch einen oder mehrere der folgenden Reste substituiert sein kann:
Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
oder R¹⁰ und R¹¹ bilden gemeinsam eine C₃-C₁₂-Alkylenkette, welche ein bis drei C₁-C₄-Alkylgruppen tragen kann;
g) oder R¹ bildet einen Rest -NH-SO₂-R¹², in dem R¹² bedeutet:
C₁-C₄-Alkyl, C₁-C₄-Halogenalkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio- und/oder einen Phenylrest tragen können;
Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio.

3. Substituierte Pyridylsalicylaldehyd- und -Salicylsäurederivate der Formel I gemäß Anspruch 1, in der mindestens zwei der Reste R¹⁵ bis R¹⁷ Wasserstoff bedeuten.

4. Substituierte Pyridylsalicylaldehyd- und -Salicylsäurederivate der Formel I gemäß Anspruch 1, in der R³ für Methoxy und X für CH, CF stehen oder R³ mit X zu einer -OCH₂CH₂- Kette verknüpft ist.

5. Substituierte Pyridylsalicylaldehyd- und -Salicylsäurederivate der Formel I gemäß Anspruch 1, in der zwei der Reste R¹⁵ bis R¹⁷ Wasserstoff und der verbleibende Rest eine C₁-C₄-Alkylgruppe darstellt.

6. Verfahren zur Herstellung von Pyridylsalicylaldehyd- bzw. -säurederivaten gemäß Anspruch 1, dadurch gekennzeichnet, daß man entsprechende aromatische ortho-Mercapto- oder ortho-Hydroxycarbonsäurederivate der Formel II 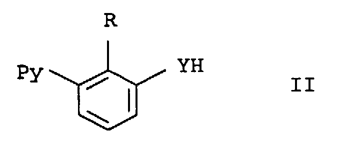 mit einem Heterocyclus der Formel III, 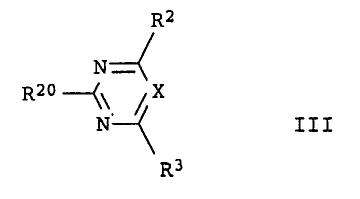 worin R²⁰ für Halogen, Alkylsulfonyl, Arylsulfonyl oder eine andere äquivalente Abgangsgruppe steht, in Gegenwart einer anorganischen oder organischen Base umsetzt.

7. Verfahren zur Herstellung von Pyridylsalicylaldehyd- bzw. -säurederivaten gemäß Anspruch 1, dadurch gekennzeichnet, daß man entsprechende Verbindungen der Formel I, wobei R¹ für den Hydroxylrest oder ein Salz des Hydroxylrestes steht, zunächst in das Halogenid oder eine andere aktivierte Form der Carbonsäure überführt und dieses dann gegebenenfalls in Gegenwart einer anorganischen oder organischen Base mit einem gegebenenfalls substituierten Alkohol, einem Azol, einem Oxim oder N-Hydroxysuccinylimin umsetzt.

8. Herbizides Mittel sowie Mittel zur Beeinflussung des Pflanzenwachstums, gekennzeichnet durch einen Gehalt an mindestens einem Pyridylsalicylaldehyd- bzw. -säurederivat der Formel I gemäß Anspruch 1 und üblichen inerten Trägerstoffen.

9. Verfahren zur Bekämpfung unerwünschten Pflanzenwuchses sowie zur Beeinflussung des Pflanzenwachstums, dadurch gekennzeichnet, daß man eine herbizid wirksame Menge eines Pyridylsalicylaldehyd- bzw. -säurederivates der Formel I gemäß Anspruch 1 auf die Pflanzen und/oder deren Lebensraum einwirken läßt.

10. Substituierte Pyridylsalicylaldehyd- und -Salicylsäurezwischenprodukte der allgemeinen Formel II, 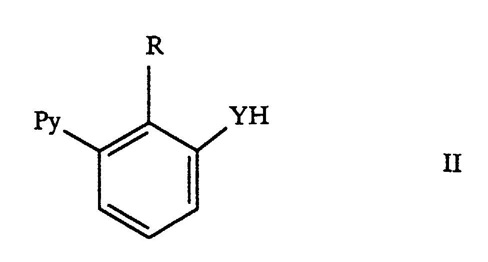 in der R eine Formylgruppe, eine Gruppe CO₂H oder einen zu CO₂H hydrolysierbaren Rest bedeutet, Y für Sauerstoff oder Schwefel steht und
Py einen in beliebiger Position verknüpften Pyridinring darstellt, der vier Substituenten R¹⁴, R¹⁵, R¹⁶ und R¹⁷ trägt:
R¹⁴
a) eine C₁-C₈-Alkoxygruppe, welche ein bis fünf Halogenatome und/oder einen der folgenden Reste tragen kann:
C₁-C₄-Alkoxy, C₁-C₄-Halogenalkoxy, C₁-C₄-Alkylthio, Cyano, C₃-C₈-Cycloalkyl oder Di-C₁-C₄-Alkylamino;
b) eine C₁-C₄-Alkylthiogruppe, welche ein bis fünf Halogenatome und/oder einen der folgenden Reste tragen kann:
C₁-C₄-Alkoxy, C₁-C₄-Halogenalkoxy, C₁-C₄-Alkylthio, Cyano, C₃-C₈-Cycloalkyl oder Di-C₁-C₄-Alkylamino;
R¹⁵, R¹⁶, R¹⁷ jeweils unabhängig voneinander Wasserstoff, Nitro, Halogen, C₁-C₈-Alkyl, C₁-C₈-Halogenalkyl sowie die bei R¹⁴ genannten Reste.

## Claims

1. A substituted pyridylsalicylaldehyde or -salicylic acid derivative of the general formula I 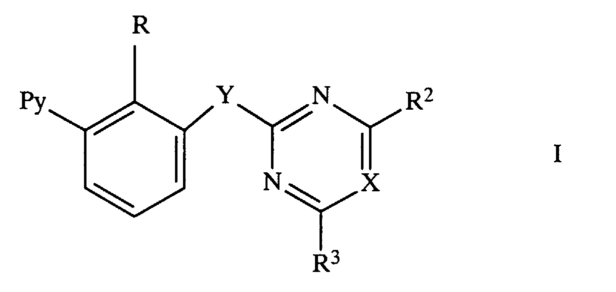 where R is a formyl group, a CO₂H group or a radical which can be hydrolyzed to give CO₂H and the other substituents have the following meanings:
R² is halogen, C₁-C₄-alkyl, C₁-C₄-haloalkyl, C₁-C₄-alkoxy, C₁-C₄-haloalkoxy or C₁-C₄-alkylthio;
X is nitrogen or CR¹³, R¹³ being hydrogen or halogen or together with R³ forming a 3- to 4-membered alkylene or alkenylene chain in which one methylene group in each case is replaced by oxygen;
R³ is halogen, C₁-C₄-alkyl, C₁-C₄-haloalkyl, C₁-C₄-alkoxy, C₁-C₄-haloalkoxy or C₁-C₄-alkylthio, or R³ is linked with R¹³ as indicated above to give a 5- or 6-membered ring;
Y is oxygen or sulfur;
Py is a pyridine ring linked in any desired position, which carries four substituents R¹⁴, R¹⁵, R¹⁶ and R¹⁷,
R¹⁴ is
a) a C₁-C₈-alkoxy group, which can carry one to five halogen atoms and/or one of the following radicals:
C₁-C₄-alkoxy, C₁-C₄-haloalkoxy, C₁-C₄-alkylthio, cyano, C₃-C₈-cycloalkyl or di-C₁-C₄-alkylamino;
b) a C₁-C₄-alkylthio group, which can carry one to five halogen atoms and/or one of the following radicals:
C₁-C₄-alkoxy, C₁-C₄-haloalkoxy, C₁-C₄-alkylthio, cyano, C₃-C₈-cycloalkyl or di-C₁-C₄-alkylamino;
R¹⁵, R¹⁶ and R¹⁷ are in each case independently of one another hydrogen, nitro, halogen, C₁-C₈-alkyl, C₁-C₈-haloalkyl and the radicals mentioned for R¹⁴.

2. A substituted pyridylsalicylaldehyde or -salicylic acid derivative of the formula I as claimed in claim 1, in which R is a group 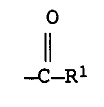 where R¹ has the following meanings:
a) hydrogen;
b) a succinylimidoxy group;
c) a 5-membered heteroaromatic linked via a nitrogen atom and containing two to three nitrogen atoms, which can carry one to two halogen atoms and/or one to two of the following radicals:
C₁-C₄-alkyl, C₁-C₄-haloalkyl, C₁-C₄-alkoxy, C₁-C₄-haloalkoxy and/or C₁-C₄-alkylthio;
d) a radical -(O)ₘ-NR⁶R⁷, where m is 0 or 1 and R⁶ and R⁷, which can be identical or different, have the following meanings:
hydrogen;
C₁-C₈-alkyl, C₃-C₆-alkenyl, C₃-C₆-alkynyl, C₃-C₈-cycloalkyl, these radicals in each case being able to carry one to five halogen atoms and/or one to two of the following groups: C₁-C₄-alkyl, C₁-C₄-alkoxy, C₃-C₆-alkenyloxy, C₃-C₆-alkynyloxy, C₁-C₄-alkylthio, C₃-C₆-alkenylthio, C₃-C₆-alkynylthio, C₁-C₄-haloalkoxy, C₁-C₄-alkylcarbonyl, C₃-C₆-alkenylcarbonyl, C₃-C₆-alkynylcarbonyl, C₁-C₄-alkoxycarbonyl, C₃-C₆-alkenyloxycarbonyl, C₃-C₆-alkynyloxycarbonyl, di-(C₁-C₄-alkyl)amino, C₃-C₈-cycloalkyl, phenyl or phenyl which is mono- or polysubstituted by halogen, nitro, cyano, C₁-C₄-alkyl, C₁-C₄-haloalkyl, C₁-C₄-alkoxy, C₁-C₄-haloalkoxy or C₁-C₄-alkylthio;
phenyl, which can be substituted by one or more of the following radicals: halogen, nitro, cyano, C₁-C₄-alkyl, C₁-C₄-haloalkyl, C₁-C₄-alkoxy, C₁-C₄-haloalkoxy or C₁-C₄-alkylthio;
R⁶ and R⁷ together are an unsubstituted or substituted C₄-C₇-alkylene chain closed to give a ring or together are an unsubstituted or substituted C₃-C₆-alkylene chain which is closed to give a ring and contains a heteroatom selected from the group consisting of oxygen, sulfur and nitrogen;
e) R¹ is additionally a group 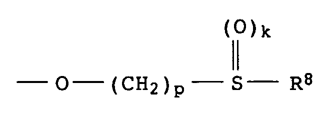 where R⁸ is C₁-C₄-alkyl, phenyl, phenyl which is mono- or polysubstituted by halogen, nitro, cyano, C₁-C₄-alkyl, C₁-C₄-haloalkyl, C₁-C₄-alkoxy, C₁-C₄-haloalkoxy or C₁-C₄-alkylthio, or C₁-C₄-haloalkyl, C₃-C₆-alkenyl or C₃-C₆-alkynyl, p assumes the values 1, 2, 3 or 4 and k assumes the values 0, 1 or 2;
f) a radical OR⁹, where R⁹ is:
i) hydrogen, an alkali metal cation, the equivalent of an alkaline earth metal cation, the ammonium cation or an organic ammonium ion;
ii) a C₃-C₈-cycloalkyl group, which can carry one to three C₁-C₄-alkyl radicals;
iii) a C₁-C₈-alkyl group, which can carry one to five halogen atoms and/or one of the following radicals:
C₁-C₄-alkoxy, C₁-C₄-alkylthio, cyano, C₁-C₄-alkylcarbonyl, C₃-C₈-cycloalkyl, C₁-C₄-alkoxycarbonyl, phenyl, or phenyl or phenoxy which is mono- or polysubstituted by halogen, nitro, cyano, C₁-C₄-alkyl, C₁-C₄-haloalkyl, C₁-C₄-alkoxy, C₁-C₄-haloalkoxy and/or C₁-C₄-alkylthio;
iv) a C₁-C₈-alkyl group, which can carry one to five halogen atoms and carries one of the following radicals: a 5-membered heteroaromatic, containing one to three nitrogen atoms, or a 5-membered heteroaromatic, containing one nitrogen atom and an oxygen or sulfur atom, which can carry one to four halogen atoms and/or one to two of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
v) a C₂-C₆-alkyl group, which in the 2-position carries one of the following radicals: C₁-C₄-alkoxyimino, C₃-C₆-alkenyloxyimino, C₃-C₆-haloalkenyloxyimino or benzyloxyimino;
vi) a C₃-C₆-alkenyl group or a C₃-C₆-alkynyl group, these groups in turn being able to carry one to five halogen atoms;
vii) a phenyl radical which can carry one to five halogen atoms and/or one to three of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
viii) a 5-membered heteroaromatic linked via a nitrogen atom and containing one to three nitrogen atoms which can carry one to two halogen atoms and/or one to two of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
ix) R⁹ is additionally a group -N=CR¹⁰R¹¹, where R¹⁰ and R¹¹, which can be identical or different, are:
C₁-C₁₂-alkyl, C₃-C₆-alkenyl, C₃-C₆-alkynyl, C₃-C₈-cycloalkyl, these radicals being able to carry a C₁-C₄-alkoxy or C₁-C₄-alkylthio radical and/or a phenyl radical;
phenyl which can be substituted by one or more of the following radicals:
halogen, nitro, cyano, C₁-C₄-alkyl, C₁-C₄-haloalkyl, C₁-C₄-alkoxy, C₁-C₄-haloalkoxy or C₁-C₄-alkylthio;
or R¹⁰ and R¹¹ together form a C₃-C₁₂-alkylene chain which can carry one to three C₁-C₄-alkyl groups;
g) or R¹ forms a radical -NH-SO₂-R¹², where R¹² is:
C₁-C₄-alkyl, C₁-C₄-haloalkyl, C₃-C₆-alkenyl, C₃-C₆-alkynyl, C₃-C₈-cycloalkyl, these radicals being able to carry a C₁-C₄-alkoxy or C₁-C₄-alkylthio radical and/or a phenyl radical;
phenyl which can be substituted by one or more of the following radicals: halogen, nitro, cyano, C₁-C₄-alkyl, C₁-C₄-haloalkyl, C₁-C₄-alkoxy, C₁-C₄-haloalkoxy or C₁-C₄-alkylthio.

3. A substituted pyridylsalicylaldehyde or -salicylic acid derivative of the formula I as claimed in claim 1, where at least two of the radicals R¹⁵ to R¹⁷ are hydrogen.

4. A substituted pyridylsalicylaldehyde or -salicylic acid derivative of the formula I as claimed in claim 1, where R³ is methoxy and X is CH or CF, or R³ is linked with X to give an -OCH₂CH₂- chain.

5. A substituted pyridylsalicylaldehyde or -salicylic acid derivative of the formula I as claimed in claim 1, where two of the radicals R¹⁵ to R¹⁷ are hydrogen and the remaining radical is a C₁-C₄-alkyl group.

6. A process for preparing pyridylsalicylaldehyde or -salicylic acid derivatives as claimed in claim 1, which comprises reacting a corresponding aromatic ortho-mercapto- or ortho-hydroxycarboxylic acid derivative of the formula II 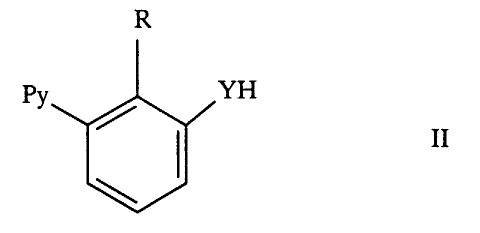 with a heterocycle of the formula III 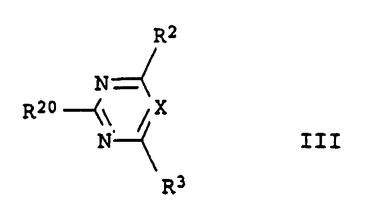 where R²⁰ is halogen, alkylsulfonyl, arylsulfonyl or another equivalent leaving group, in the presence of an inorganic or organic base.

7. A process for preparing pyridylsalicylaldehyde or -salicylic acid derivatives as claimed in claim 1, which comprises converting corresponding compounds of the formula I where R¹ is the hydroxyl radical or a salt of the hydroxyl radical first to the halide or another activated form of the carboxylic acid and then reacting this with an unsubstituted or substituted alcohol, an azole, an oxime or N-hydroxysuccinylimine, if appropriate in the presence of an inorganic or organic base.

8. A herbicidal composition or composition for influencing plant growth, which contains at least one pyridylsalicylaldehyde or -salicylic acid derivative of the formula I as claimed in claim 1 and customary inert carriers.

9. A process for controlling undesired plant growth and for influencing plant growth, which comprises allowing a herbicidally active amount of a pyridylsalicylaldehyde or -salicylic acid derivative of the formula I as claimed in claim 1 to act on the plants and/or their environment.

10. A substituted pyridylsalicylaldehyde or -salicylic acid intermediate of the general formula II 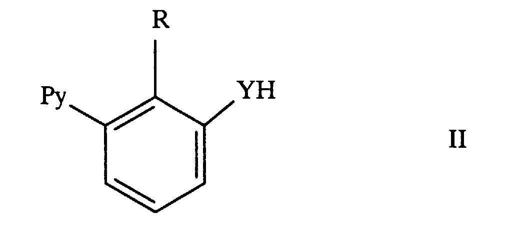 where R is a formyl group, a CO₂H group or a radical which can be hydrolyzed to CO₂H, Y is oxygen or sulfur and
Py is a pyridine ring linked in any desired position, which carries four substituents R¹⁴, R¹⁵, R¹⁶ and R¹⁷:
R¹⁴ is
a) a C₁-C₈-alkoxy group, which can carry one to five halogen atoms and/or one of the following radicals:
C₁-C₄-alkoxy, C₁-C₄-haloalkoxy, C₁-C₄-alkylthio, cyano, C₃-C₈-cycloalkyl or di-C₁-C₄-alkylamino;
b) a C₁-C₄-alkylthio group, which can carry one to five halogen atoms and/or one of the following radicals:
C₁-C₄-alkoxy, C₁-C₄-haloalkoxy, C₁-C₄-alkylthio, cyano, C₃-C₈-cycloalkyl or di-C₁-C₄-alkylamino;
R¹⁵, R¹⁶ and R¹⁷ are in each case independently of one another hydrogen, nitro, halogen, C₁-C₈-alkyl, C₁-C₈-haloalkyl and the radicals mentioned for R¹⁴.

## Revendications

1. Dérivés substitués de l'aldéhyde et de l'acide pyridylsalicyliques répondant à la formule générale I 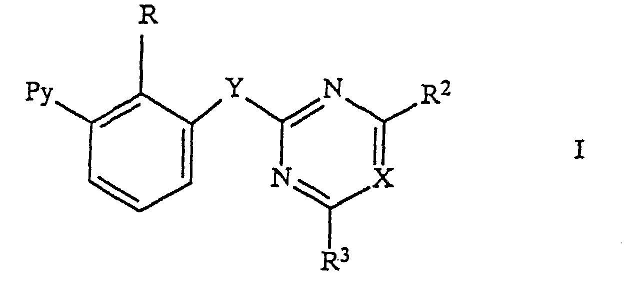 dans laquelle R représente un groupe formyle, un groupe CO₂H ou un groupe hydrolysable en un groupe CO₂H et les autres symboles ont les significations suivantes :
R² : un halogène, un groupe alkyle en C1-C4, halogénoalkyle en C1-C4, alcoxy en C1-C4, halogénoalcoxy en C1-C4 ou alkylthio en C1-C4 ;
X : l'azote ou un groupe CR¹³ dans lequel R¹³ représente l'hydrogène ou un halogène ou bien, avec R³, une chaîne alkylène ou alcénylène à trois ou quatre chaînons et dans laquelle un groupe méthylène est remplacé par l'oxygène ;
R³ : un halogène, un groupe alkyle en C1-C4, halogénoalkyle en C1-C4, alcoxy en C1-C4, halogénoalcoxy en C1-C4, alkylthio en C1-C4, ou bien R³ est relié avec R¹³ comme indiqué ci-dessus, formant un cycle à cinq ou six chaînons ;
Y : l'oxygène ou le soufre ;
Py : un cycle pyridine relié dans une position quelconque et qui porte quatre substituants R¹⁴, R¹⁵, R¹⁶ et R¹⁷ ;
R¹⁴ :
a) un groupe alcoxy en C1-C8 qui peut porter un à cinq atomes d'halogènes et/ou un des groupes suivants :
alcoxy en C1-C4, halogénoalcoxy en C1-C4, alkylthio en C1-C4, cyano, cycloalkyle en C3-C8 ou di-(alkyle en C1-C4)amino;
b) un groupe alkylthio en C1-C4 qui peut porter un à cinq atomes d'halogènes et/ou un des groupes suivants :
alcoxy en C1-C4, halogénoalcoxy en C1-C4, alkylthio en C1-C4, cyano, cycloalkyle en C3-C8 ou di-(alkyle en C1-C4)amino ;
R¹⁵, R¹⁶, R¹⁷ :
chacun, indépendamment les uns des autres : l'hydrogène, un groupe nitro, un halogène, un groupe alkyle en C1-C8, halogénoalkyle en C1-C8 ou l'un des groupes mentionnés en référence à R¹⁴.

2. Dérivés substitués de l'aldéhyde et de l'acide pyridylsalicyliques de formule I selon la revendication 1, dans laquelle R représente un groupe 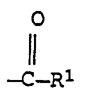 dans lequel R¹ a l'une des significations suivantes :
a) l'hydrogène,
b) un groupe succinylimidoxy ;
c) un groupe hétéroaromatique à cinq chaînons, relié par l'intermédiaire d'un atome d'azote et contenant deux à trois atomes d'azote, qui peut porter un à deux atomes d'halogènes et/ou un à deux des groupes suivants :
alkyle en C1-C4, halogénoalkyle en C1-C4, alcoxy en C1-C4, halogénoalcoxy en C1-C4 et/ou alkylthio en C1-C4 ;
d) un groupe -(O)m-NR⁶R⁷ dans lequel m est égal à 0 ou 1 et R⁶ et R⁷, qui peuvent avoir des significations identiques ou différentes, représentent chacun :
l'hydrogène ;
un groupe alkyle en C1-C8, alcényle en C3-C6, alcynyle en C3-C6, cycloalkyle en C3-C8, ces groupes pouvant porter chacun un à cinq atomes d'halogènes et/ou un à deux des groupes suivants : alkyle en C1-C4, alcoxy en C1-C4, alcényloxy en C3-C6, alcynyloxy en C3-C6, alkylthio en C1-C4, alcénylthio en C3-C6, alcynylthio en C3-C6, halogénoalcoxy en C1-C4, (alkyle en C1-C4)carbonyle, (alcényle en C3-C6)carbonyle, (alcynyle en C3-C6)carbonyle, (alcoxy en C1-C4)carbonyle, (alcényloxy en C3-C6)carbonyle, (alcynyloxy en C3-C6)carbonyle, di-(alkyle en C1-C4)amino, cycloalkyle en C3-C8, phényle, phényle portant un ou plusieurs substituants halogéno, nitro, cyano, alkyle en C1-C4, halogénoalkyle en C1-C4, alcoxy en C1-C4, halogénoalcoxy en C1-C4 ou alkylthio en C1-C4 ;
phényle, qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4 ;
R⁶ et R⁷ forment ensemble une chaîne alkylène en C4-C7 fermée en un cycle et éventuellement substituée ou une chaîne alkylène en C3-C6 fermée en un cycle, éventuellement substituée, et contenant un hétéroatome choisi parmi l'oxygène, le soufre et l'azote ;
e) R¹ peut en outre représenter un groupe de formule 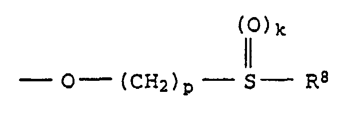 dans laquelle R⁸ représente un groupe alkyle en C1-C4, phényle, phényle portant un ou plusieurs substituants halogéno, nitro, cyano, alkyle en C1-C4, halogénoalkyle en C1-C4, alcoxy en C1-C4, halogénoalcoxy en C1-C4 ou alkylthio en C1-C4, un groupe halogénoalkyle en C1-C4, alcényle en C3-C6 ou alcynyle en C3-C6, p est égal à 1, 2, 3 ou 4 et k est égal à 0, 1 ou 2 ;
f) un groupe OR⁹ dans lequel R⁹ représente :
i) l'hydrogène, un cation de métal alcalin, un équivalent de cation de métal alcalino-terreux, le cation ammonium ou un ion ammonium organique ;
ii) un groupe cycloalkyle en C3-C8 qui peut porter un à trois groupes alkyle en C1-C4 ;
iii) un groupe alkyle en C1-C8 qui peut porter un à cinq atomes d'halogènes et/ou un des groupes suivants :
alcoxy en C1-C4, alkylthio en C1-C4, cyano, (alkyle en C1-C4)carbonyle, cycloalkyle en C3-C8, (alcoxy en C1-C4)carbonyle, phényle, phényle ou phénoxy portant un ou plusieurs substituants halogéno, nitro, cyano, alkyle en C1-C4, halogénoalkyle en C1-C4, alcoxy en C1-C4, halogénoalcoxy en C1-C4 et/ou alkylthio en C1-C4 ;
iv) un groupe alkyle en C1-C8 qui peut porter un à cinq atomes d'halogènes et porte un des groupes suivants : un groupe hétéroaromatique à cinq chaînons contenant un à trois atomes d'azote, ou un groupe hétéroaromatique à cinq chaînons contenant un atome d'azote et un atome d'oxygène ou de soufre, qui peuvent porter un à quatre atomes d'halogènes et/ou un à deux des groupes suivants : nitro, cyano, alkyle en C1-C4, halogénoalkyle en C1-C4, alcoxy en C1-C4, halogénoalcoxy en C1-C4 et/ou alkylthio en C1-C4 ;
v) un groupe alkyle en C2-C6 qui porte en position 2 l'un des groupes suivants : alcoxyimino en C1-C4, alcényloxyimino en C3-C6, halogénoalcényloxyimino en C3-C6 ou benzyloxyimino ;
vi) un groupe alcényle en C3-C6 ou alcynyle en C3-C6 qui peut lui-même porter un à cinq atomes d'halogènes ;
vii) un groupe phényle qui peut porter un à cinq atomes d'halogènes et/ou un à trois des groupes suivants : nitro, cyano, alkyle en C1-C4, halogénoalkyle en C1-C4, alcoxy en C1-C4, halogénoalcoxy en C1-C4 et/ou alkylthio en C1-C4 ;
viii) un groupe hétéroaromatique à cinq chaînons relié par l'intermédiaire d'un atome d'azote, qui contient un à trois atomes d'azote et qui peut porter un à deux atomes d'halogènes et/ou un à deux des groupes suivants : nitro, cyano, alkyle en C1-C4, halogénoalkyle en C1-C4, alcoxy en C1-C4, halogénoalcoxy en C1-C4 et/ou alkylthio en C1-C4 ;
ix) R⁹ peut en outre représenter un groupe -N=CR¹⁰R¹¹ dans lequel R¹⁰ et R¹¹, qui peuvent avoir des significations identiques ou différentes, représentent chacun : un groupe alkyle en C1-C12, alcényle en C3-C6, alcynyle en C3-C6, cycloalkyle en C3-C8, ces groupes pouvant porter un groupe alcoxy en C1-C4, alkylthio en C1-C4 et/ou un groupe phényle ;
le groupe phényle, qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4 ;
ou bien R¹⁰ et R¹¹ forment ensemble une chaîne alkylène en C3-C12 qui peut porter un à trois groupes alkyle en C1-C4 ;
g) ou bien R¹ représente un groupe -NH-SO₂-R¹² dans lequel R¹² représente :
un groupe alkyle en C1-C4, halogénoalkyle en C1-C4, alcényle en C3-C6, alcynyle en C3-C6, cycloalkyle en C3-C8, ces groupes pouvant porter un groupe alcoxy en C1-C4, alkylthio en C1-C4 et/ou phényle ;
un groupe phényle qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4.

3. Dérivés substitués de l'aldéhyde et de l'acide pyridylsalicyliques de formule I selon la revendication 1 dans laquelle au moins deux des symboles R¹⁵ à R¹⁷ représentent l'hydrogène.

4. Dérivés substitués de l'aldéhyde et de l'acide pyridylsalicyliques de formule I selon la revendication 1, dans laquelle R³ représente un groupe méthoxy et X représente CH, CF, ou bien R³ forme avec X une chaîne -OCH₂CH₂-.

5. Dérivés substitués de l'aldéhyde et de l'acide pyridylsalicyliques de formule I selon la revendication 1, dans laquelle deux des symboles R¹⁵ à R¹⁷ représentent l'hydrogène et le troisième un groupe alkyle en C1-C4.

6. Procédé de préparation des dérivés de l'aldéhyde et de l'acide pyridylsalicyliques selon la revendication 1, caractérisé par le fait que l'on fait réagir un dérivé d'acide orthomercapto- ou ortho-hydroxycarboxylique aromatique correspondant de formule II 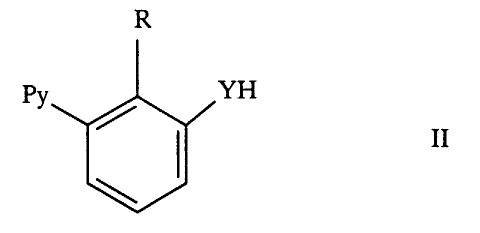 avec un dérivé hétérocyclique de formule III, 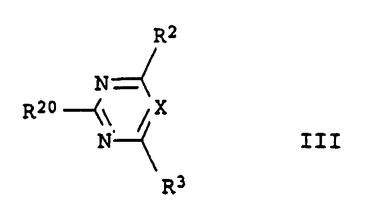 dans laquelle R²⁰ représente un halogène, un groupe alkylsulfonyle, arylsulfonyle ou un autre groupe éliminable équivalent en présence d'une base organique ou minérale.

7. Procédé de préparation des dérivés de l'aldéhyde et de l'acide pyridylsalicyliques selon la revendication 1, caractérisé par le fait que l'on convertit d'abord en l'halogénure ou en une autre forme activée de l'acide carboxylique les composés correspondants de formule I dans laquelle R¹ représente le groupe hydroxy éventuellement à l'état de sel et on fait ensuite réagir, éventuellement en présence d'une base organique ou minérale, avec un alcool, un azole, une oxime ou la N-hydroxysuccinylimine éventuellement substitutés.

8. Produit herbicide et produit pour agir sur la croissance des végétaux, caractérisé par le fait qu'il contient au moins un dérivé d'aldéhyde ou d'acide pyridylsalicylique de formule I selon la revendication 1, et des véhicules inertes usuels.

9. Procédé pour combattre les croissances de végétaux indésirables et pour agir sur la croissance des végétaux, caractérisé par le fait que l'on fait agir sur les végétaux et/ou leur habitat une quantité herbicide efficace d'un dérivé de l'aldéhyde ou de l'acide pyridylsalicylique de formule I selon la revendication 1.

10. Produits intermédiaires de la préparation d'aldéhydes et d'acides pyridylsalicyliques substitués, de formule générale II 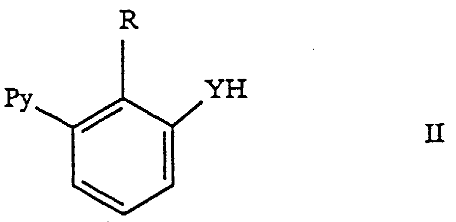 dans laquelle R représente un groupe formyle, un groupe CO₂H ou un groupe hydrolysable en un groupe CO₂H, Y représente l'oxygène ou le soufre et
Py représente un cycle pyridine relié dans une position quelconque et qui porte quatre substituants R¹⁴, R¹⁵, R¹⁶ et R¹⁷ :
R¹⁴ représente
a) un groupe alcoxy en C1-C8 qui peut porter un à cinq atomes d'halogènes et/ou un des groupes suivants :
alcoxy en C1-C4, halogénoalcoxy en C1-C4, alkylthio en C1-C4, cyano, cycloalkyle en C3-C8 ou di-(alkyle en C1-C4)amino ;
b) un groupe alkylthio en C1-C4 qui peut porter un à cinq atomes d'halogènes et/ou un des groupes suivants :
alcoxy en C1-C4, halogénoalcoxy en C1-C4, alkylthio en C1-C4, cyano, cycloalkyle en C3-C8 ou di-(alkyle en C1-C4)amino ;
R¹⁵, R¹⁶, R¹⁷ représentent
chacun, indépendamment les uns des autres, l'hydrogène, un groupe nitro, un halogène, un groupe alkyle en C1-C8, halogénoalkyle en C1-C8 ou l'un des groupes mentionnés en référence à R¹⁴.
